# EUROPEAN PATENT APPLICATION

(11) **EP 4 825 371 A1**
(43) Date of publication of application: **21.10.2026**
(21) Application number: 26172363.9
(22) Date of filing: 14.04.2026
(51) Int. Cl.: A61K 9/00, A61K 47/10, A61K 47/26, A61K 47/36, A61K 47/42

(54) **CHEWABLE GUMMY-GEL FORMULATIONS AND METHODS OF MAKING SAME**

(30) Priority: 14.04.2025 US 202563788589 P
(71) Applicant: Aspire LLC, Somerset, NJ 08873 (US)
(72) Inventor: Zhang, Zhang, Somerset, 08873 (US); Pai, Madhav, Somerset, 08873 (US)
(74) Representative: EIP

(57) **Abstract**

The present disclosure provides chewable gel compositions that provide pleasant mouth feel throughout, while maintaining the thermal and chemical stability of active agents over long-term storage. Such chewable gel compositions can comprise a gummy fill material encapsulated by a chewable soft gel capsule. The present disclosure also provides methods of making chewable gel compositions using a rotary die process.

## Description

### RELATED APPLICATIONS

This application claims the benefit of priority to U.S. Provisional Application No. 63/788,589 filed on April 14, 2025, the entire contents of which are incorporated herein by reference.

### BACKGROUND

Chewable dosage forms have important applications in administering active agents for medicine and dietary supplements, since a significant portion of the population prefers chewing to swallowing, or have difficulty swallowing medicaments. However, despite this preference, the acceptance of chewable dosage forms is limited on account of their unpleasant mouth feel. In addition, chewable dosage forms need to be able to maintain the stability of the active agent during manufacture and over long-term storage.

### SUMMARY

In one aspect, the present invention provides a chewable gel composition comprising a chewable fill material encapsulated by a chewable gel shell, wherein the chewable fill material comprises one or more gummy-gel forming agents present at a total of about 0.1-40% (w/w) of the chewable fill material. In some embodiments, the one or more gummy-gel forming agents is present at a total of about 0.1%-25%, 0.1%-20%, 0.1%-15%, 0.1%-13%, 2%-25%, 2%-20%, 5%-25%, 5%-24%, 6%-24%, 7%-24%, 2%-15%, 2%-13%, 4%-15%, 4%-13%, 5%-15%, 5%-13%, 7%-15%, 7%-13%, 8%-15%, or 8%-13% (w/w) of the chewable fill material. In some embodiments, the one or more gummy-gel forming agents comprises a monomer, an oligomer, a polymer, a copolymer or combinations thereof. In some embodiments, the one or more gummy-gel forming agents comprises gelatin, starch, pectin, carbomer, poloxamer or a combination thereof. In some embodiments, the one or more gummy-gel forming agents comprises gelatin.

In some embodiments, the chewable fill material further comprises one or more crystallization inhibitors. In some embodiments, the one or more crystallization inhibitors comprises glucose syrup, corn syrup, povidone, fructose syrup, glycerin, hydrogenated starch hydrolysate, or a combination thereof. In some embodiments, the one or more crystallization inhibitors are present at a total of about 30-90% (w/w) of the chewable fill material. In some embodiments, the one or more crystallization inhibitors are present at a total of about 30%-85%, 30%-80%, 30%-70%, 40%-75%, 30%-60%, 35%-60%, 40%-60% or 40%-55%, (w/w) of the chewable fill material.

In some embodiments, the chewable fill material further comprises one or more pH adjusting agents. In some embodiments, the one or more pH adjusting agents are present at a total of about 0.01 - 10% (w/w) or 0.1-5% (w/w) of the chewable fill material. In some embodiments, the one or more pH adjusting agents are present at a total of about 0.1-3%, 0.2-3%, or 0.2-2% (w/w) of the chewable fill material. In some embodiments, the one or more pH adjusting agents comprise citric acid, ascorbic acid, lactic acid, sodium citrate, sodium bicarbonate, malic acid, or combinations thereof. In some embodiments, the one or more pH adjusting agents comprise at least one of citric acid or malic acid.

In some embodiments, the chewable fill material further comprises one or more sweeteners. In some embodiments, the one or more sweeteners are present at a total of about 0.1-35% (w/w) of the chewable fill material. In some embodiments, the one or more sweeteners are present at a total of about 4-34%, 5-33%, 5-32%, or 5-20% (w/w) of the chewable fill material. In some embodiments, the one or more sweeteners comprise sucrose, glycerin, glucose, hydrogenated starch hydrolysate, sucralose, xylitol, or combinations thereof.

In some embodiments, the chewable fill material further comprises one or more flavoring agents. In some embodiments, the one ore more flavoring agents are present at a total of about 0.1-5% (w/v) of the chewable fill material. In some embodiments, the one or more flavoring agents are present at a total of about 0.5-3%, 0.7-2%, or 1-1.6% (w/v) of the chewable fill material. In some embodiments, the one ore more flavoring agents comprise natural flavors and/or artificial flavors.

In some embodiments, the chewable fill material further comprises one or more solvents. In some embodiments, the one or more solvents are present at a total of about 0.1-15% (w/w) of the chewable fill material. In some embodiments, the one or more solvents are present at a total of about 0.5-13%, 1-12%, or 1.5-10% (w/w) of the chewable fill material. In some embodiments, the one or more solvents comprises water, oil, propylene glycol, polyethylene glycol (PEG), medium-chain triglycerides (MCT) oil, or combinations thereof.

In some embodiments, wherein the chewable fill material further comprises one or more lipids. In some embodiments, the one or more lipids are present at a total of about 0.1-20% (w/w) of the chewable fill material. In some embodiments, the one or more lipids are present at a total of about 0.5-15%, 2-15%, 5-15%, or 5-12% (w/w) of the chewable fill material. In some embodiments, the one or more lipids comprises MCT oil, olive oil, sunflower oil, fish oil, krill oil, flaxseed oil, hemp oil, chia oil, evening primrose oil, borage oil, black cumin oil, avocado oil, eicosapentaenoic acid (EPA), docosahexaenoic acid (DHA), beeswax, carnauba wax, rice bran, candelilla, or combinations thereof. In some embodiments, the lipid comprises MCT oil.

In some embodiments, the chewable fill material further comprises an active ingredient. In some embodiments, the active ingredient is a pharmaceutical compound, a nutraceutical compound, or a dietary supplement. In some embodiments, the active ingredient comprises dextromethorphan HBr, doxylamine succinate, diphenhydramine HCl, ubiquinol, creatine, lutein, zeaxanthin, ibuprofen, guaifenesin, acetaminophen, triprolidine HCl, Coenzyme Q10 (CoQ10), or combinations thereof.

In some embodiments, the chewable fill material is a gummy composition.

In some embodiments, the chewable fill material has a viscosity of about 300-16,000 cap at a temperature of about 35-45°C, optionally at 40 °C, prior to encapsulation by the chewable gel shell. In some embodiments, the chewable fill material has a viscosity of about 350-15,000 cP, 350-12,500 cP, 400-10,000 cP, 400-9,000 cP, 400-8,000 cP, 500-8,00 cP, 400-7,000 cP, 400-6,000 cP, 400-7,000 cP, 400-6,000 cP, 400-5,000 cP, 400-4,000 cP, 400-3,000 cP, 400-2,000 cP, 400-1,000 cP, or 450-900 cP at a temperature of about 40 °C, prior to encapsulation by the chewable gel shell. In some embodiments, wherein encapsulation is through a rotary die process.

In some embodiments, the chewable gel shell comprises one or more of gelling agents, plasticizers, anti-tacking agents, softening agents, sweeteners, flavoring agents, colorants, preservatives or combinations thereof.

In another aspect, the present invention provides a chewable gel composition comprising a chewable fill material encapsulated by a chewable gel shell, wherein the chewable fill material comprises: 3-15% (w/w) gelatin; and 40-90% (w/w) of one or more crystallization inhibitors. In some embodiments, the chewable composition further comprises about 1-3% of one or more pH adjusting agent. In some embodiments, the pH adjusting agent is at least one of malic acid or citric acid, optionally present at a total of about 0.1-3% (w/w). In some embodiments, the chewable fill material comprises about 6-13% (w/w) gelatin. In some embodiments, the crystallization inhibitor is selected from the group consisting of corn syrup, glycerin, glucose syrup, fructose syrup, hydrogenated starch hydrolysate and a combination thereof. In some embodiments, the chewable fill material further comprises povidone, propylene glycol, xylitol and/or a flavoring agent. In some embodiments, the chewable fill material further comprises a lipid, for example, about 5-15% (w/w) MCT oil. In some embodiments, the chewable fill material has a viscosity of about 300-16,000 cP at a temperature of about 35-45°C, optionally at 40 °C, prior to encapsulation by the chewable gel shell.

In another aspect, the present invention provides a method of making a chewable gel composition, comprising: (a) preparing a chewable fill material mixture comprising one or more gummy-gel forming agents, one or more crystallization inhibitors and a pH adjusting agent; (b) incubating the mixture of step (a) at a fixed temperature for a time period sufficient to achieve a uniform mixture; and (c) encapsulating the uniform mixture of step (b) by a chewable gel shell using a rotary die process. In some embodiments, step (b) comprises incubating the mixture at a temperature ranging from 25-70 °C. In some embodiments, step (b) comprises maintaining the mixture at a fixed temperature for a time period of about 0.5-48 hours. In some embodiments, the uniform mixture of step (b) has a viscosity of about 300-16,000 cP at a temperature of about 35-45°C, optionally at 40 °C. In some embodiments, the uniform mixture of step (b) has a viscosity of about 350-15,000 cP, 350-12,500 cP, 400-10,000 cP, 400-9,000 cP, 400-8,000 cP, 500-8,000 cP, 400-7,000 cP, 400-6,000 cP, 400-7,000 cP, 400-6,000 cP, 400-5,000 cP, 400-4,000 cP, 400-3,000 cP, 400-2,000 cP, 400-1,000 cP, or 450-900 cP at a temperature of about 40 °C.

In some embodiments, the present invention provides a chewable gel composition made by the methods as provided herein.

### BRIEF DESCRIPTION OF THE DRAWINGS

**Figure 1A** is plot showing an HPLC chromatogram for Formula 1 containing triprolidine HCl.
**Figure 1B** is a plot showing an HPLC chromatogram for Formula 2 containing triprolidine HCl.
**Figure 2** is a plot showing the change in water content in chewable gel compositions over time.

### DETAILED DESCRIPTION

Chewable dosage forms include compositions such as soft capsules which are formed of a shell encapsulating a fill material. The fill material is usually a liquid, suspension or paste, which is released in the mouth when the user chews the shell. Although chewable soft capsules are an effective dosage form, their acceptance has been limited due to disadvantages such as an unpleasant mouth feel when the fill is released in the user's mouth, and the difference in texture and mouth-feel between the soft capsule and the fill material.

Gummy compositions are another chewable dosage form, which have the advantage of providing a pleasant mouth feel on account of their gummy-like texture. However, gummy dosage forms suffer from the drawback of being susceptible to external environmental factors, *e.g.*, temperature, humidity, oxygen degradation, *etc*. In the absence of measures for proper preservation, gummies may lose potency, and their appearance and palatability could be impaired over time. Moreover, the gummy manufacturing process usually employs high temperatures (in excess of 100 °C) to boil off excess water and prevent the gummies from setting too quickly. This presents a significant risk of thermal degradation for heat sensitive active compounds in gummy dosage forms.

A rotary die process provides a more gentle method involving lower temperatures to encapsulate chewable fill materials. However, the composition of a chewable fill material affects its rheological profile, (*e.g.*, viscosity), which in turn dictates its laminar flow characteristics during a rotary die process, such as during the injection phase. For example, proper viscosity ensures the fill material transitions seamlessly through the injection pump and into the pocket formed by the gelatin or polymer ribbons. Formulations with a high viscosity require significantly higher head pressure for displacement, and can lead to cavitation within the pump, resulting in inconsistent volumetric dosing and weight variation. Furthermore, highly viscous fills can interfere with the thermal sealing of the die, leading to leaky capsules or compromised seam integrity. On the other hand, insufficient viscosity may lead to premature splashing or improper displacement (*e.g.*, during the high-speed injection stroke of a rotary die process), potentially resulting in air entrapment. Typically, optimal results are achieved in a rotary die process within a narrow operational window for viscosity. In addition to its importance during mechanical processing of the fill mixture, viscosity is also a primary determinant of the mouthfeel and chewability of the fill material in the gummy end product. A formulation with insufficient viscosity fails to provide the structural resistance and chew characteristics required for a chewable delivery format, often resulting in an unpleasant liquid-like sensation.

Balancing these various factors including mouthfeel, texture, manufacturing stability, storage stability, desirable viscosity and amenability to manufacturing processes is particularly challenging.

The present disclosure provides a chewable dosage form that possesses a pleasant mouth feel throughout, while simultaneously maintaining thermal and chemical stability of the active ingredient, at least in part, by providing compositions and methods of manufacturing a chewable dosage form comprising a soft capsule encapsulating a gummy/jelly fill material. The present disclosure is based, at least in part, on the surprising formulation of gummy compositions encapsulated in soft gel capsules so as to provide excellent mouth feel, and which can be manufactured at lower temperatures using a rotary die process. In certain embodiments, the gummy compositions comprise low levels (*e.g.* less than 20%, 15%, 10%, 9% *etc*.) of gummy-gel forming agents. The formulations of the gummy compositions disclosed herein have rheological profiles which are advantageous for the processability of the gummy fill material as well as end-product quality.

Accordingly, the present disclosure provides chewable gel compositions comprising a chewable fill material encapsulated by a chewable gel shell, wherein the fill material is a gummy/gel composition. Also provided are methods of preparing a chewable gel composition, comprising: preparing a chewable fill material mixture, incubating the mixture at a fixed temperature for a time period sufficient to achieve a uniform mixture, and encapsulating the uniform mixture by a chewable gel shell using a rotary die process. The inventions of the present disclosure provide the following advantages and benefits over conventional chewable dosage forms:
a. The gummy-gel fill material of the present disclosure reduces the difference in texture and provides a better consistency between the outer capsule and the inner fill material, which significantly improves the mouth feel and palatability of the chewable product.
b. The fill mixtures disclosed herein are characterized by viscosity falling within the operational window of a rotary die process, which render them amenable to encapsulation using a softgel rotary die process.
c. By encapsulating the gummy dosage form in a protective soft shell, the chewable gummy-gel formulations disclosed herein reduce exposure of the active compound to harmful environmental factors such as temperature, humidity, oxygen degradation, *etc*. This encapsulation prevents loss of potency, appearance and palatability over time. During a rotary die process, the fill material is encapsulated at temperatures of about 25-65 °C, which is suitable for active compounds that are susceptible to thermal degradation. This method of manufacture overcomes the detrimental effects of the high temperatures used in the conventional gummy manufacturing process.
Various aspects of the present disclosure are described in further detail in the following subsections.

### Definitions

Unless defined otherwise, all technical and scientific terms used herein have the same meaning as commonly understood by one of ordinary skill in the art to which the invention pertains.

As used in this specification and the appended claims, the singular forms "a," "an," and "the" include plural references unless the content clearly dictates otherwise.

The articles "a" and "an" are used herein to refer to one or to more than one (*e.g.*, to at least one) of the grammatical object of the article. By way of example, "an element" means one element or more than one element, *e.g.*, a plurality of elements.

The use of the alternative (*e.g.*, "or") should be understood to mean either one, both, or any combination thereof of the alternatives. The term "or" is used herein to mean, and is used interchangeably with, the term "and/or," unless context clearly indicates otherwise.

As used herein, the term "about," when referring to a measurable value such as an amount, a temporal duration, and the like, is meant to encompass variations of ±20%, or ±10%, more preferably ±5%, even more preferably ±1%, and still more preferably ±0.1% from the specified value, as such variations are appropriate to perform the disclosed methods.

As used herein, any concentration range, percentage range, ratio range, or integer range is to be understood to include the value of any integer within the recited range and, when appropriate, fractions thereof (such as one tenth and one hundredth of an integer), unless otherwise indicated.

As used herein, "comprise," "comprising," "comprises," and "comprised of" are meant to be synonymous with "include," "including," "includes," or "contain," "containing," "contains" and are inclusive or open-ended terms that specifies the presence of what follows, *e.g.*, component and do not exclude or preclude the presence of additional, non-recited components, features, element, members, steps, known in the art or disclosed therein. By way of example, the term "including" is used herein to mean, and is used interchangeably with, the phrase "including but not limited to."

As used herein, the terms "such as," "for example," and the like are intended to refer to exemplary embodiments and not to limit the scope of the present disclosure.

Recitation of ranges of values herein are merely intended to serve as a shorthand method of referring individually to each separate value recited or falling within the range, unless otherwise indicated herein, and each separate value is incorporated into the specification as if it were individually recited.

The term "at least" prior to a number or series of numbers is understood to include the number adjacent to the term "at least", and all subsequent numbers or integers that could logically be included, as clear from context. When at least is present before a series of numbers or a range, it is understood that "at least" can modify each of the numbers in the series or range.

As used herein, "no more than" or "less than" is understood as the value adjacent to the phrase and logical lower values or integers, as logical from context, to zero. When "no more than" is present before a series of numbers or a range, it is understood that "no more than" can modify each of the numbers in the series or range. As used herein, ranges include both the upper and lower limit.

As used herein, the various forms of the term "modulate" or "alter" are intended to include stimulation, activation, and/or enhancement (*e.g.*, increasing or upregulating a particular response, level, or activity) and inhibition (*e.g.*, decreasing or downregulating a particular response, level, or activity).

As used herein, the term "drug delivery system" refers to a composition for administering an active agent to a subject in need thereof, such as a human or an animal. In some embodiments, the drug delivery system comprises a chewable gel composition of the present disclosure. As used herein, the term "conventional drug delivery system" refers to a drug delivery system that does not include the chewable gel composition of the present disclosure.

The terms "decrease," "reduced," "reduction," or "inhibit" are all used herein to mean a decrease by a statistically significant amount. In some embodiments, the terms "reduced," "reduction," "decrease," or "inhibit" can mean a decrease by at about least 5% as compared to a reference level, for example a decrease by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99% or more as compared to a reference level. In some embodiments, the terms can represent a 100% decrease, *e.g.*, a non-detectable level as compared to a reference level.

The terms "increased," "increase," "enhance," or "activate" are all used herein to mean an increase by a statically significant amount. In some embodiments, the terms "increased," "increase," "enhance," or "activate" can mean an increase of at least about 5% as compared to a reference level, for example an increase of at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, at least about 100%, at least about 250%, at least about 500%, or at least about 1000% or more. In some embodiments, the terms can represent an increase up to and including a 100% increase as compared to a reference level, or at least about a 2-fold, or at least about a 3-fold, or at least about a 4-fold, or at least about a 5-fold, or at least about a 10-fold increase, or at least about a 100-fold increase, or at least about a 1000-fold increase, or any increase between 2-fold and 1000-fold or greater as compared to a reference level. In the context of a marker or symptom, an "increase" is a statistically significant increase in such level.

As used herein, "substantially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein, the term "administering" to a subject includes dispensing, delivering or applying a composition as described herein to a subject by any suitable route for delivery of the composition to the subject. Exemplary modes of administration include delivery by the oral route.

The term "solubility" as used herein, refers to the amount of a substance that will dissolve in a given amount of another substance. Typically, solubility is expressed as the number of parts by weight dissolved by 100 parts of solvent at a specified temperature and pressure or as percent by weight or by volume.

As used herein, the term "unit dosage form" or "dosage form" refers to physically discrete units suitable as unitary dosages for subjects, *e.g.*, human subjects, each unit containing a predetermined quantity of an active agent and/or a composition comprising an active agent (*e.g.*, a composition described herein), calculated in an amount sufficient to produce the desired biological effect in association with a pharmaceutically acceptable diluent, carrier, or vehicle.

As used herein, the term "oral dosage form" refers to a unit dosage form that may be orally administered. In some embodiments, the compositions described herein may be manufactured and/or administered as oral dosage forms. In some embodiments, the oral dosage forms may comprise an amount (*e.g.*, a therapeutically effective amount and/or a prophylactically effective amount) of a composition described herein.

In some embodiments, the oral dosage forms may further comprise one or more agents selected from the group consisting of a sweetener, such as sucrose, glycerin, glucose, hydrogenated starch hydrolysate, sucralose and the like; a preservative, such as potassium sorbate, sodium benzoate, parabens, and the like; a natural flavor; an artificial flavor; a taste masking agent, such as citric acid, sodium bicarbonate, and the like; a viscosity builder, such as xanthan gum, starch, guar gum, hypromellose, also known as hydroxypropyl methylcellulose (HPMC), and the like; a colorant; and combinations thereof.

### Chewable Gummy-Gel Compositions

According to one aspect, the present disclosure provides chewable gummy-gel compositions comprising a gummy-gel fill material encapsulated in a chewable gel shell.

### Fill Material

The compositions of the present disclosure comprise a chewable fill material, *e.g.*, a gummy fill material. In some embodiments, the chewable fill material is a gummy composition.

As used herein, the term "gummy" refers to a composition that is characterized by its chewy texture and mouth feel. Gummy bears, gummy worms and other gummies characterized by their elastic, firm, and bouncy textures are known in the art, and one of ordinary skill in the art would understand that the term "gummy" refers to a composition having such a texture and mouth feel.

In some embodiments, the chewable fill materials of the disclosure comprise a gummy-gel forming agent. In some embodiments, the chewable fill materials comprise a crystallization inhibitor. In some embodiments, the chewable fill material further comprises one or more of a pH adjusting agent, a sweetener, a flavor, a taste-masking component, a solvent, a lipid, an active component, water, and combinations thereof.

The chewable fill materials of the present disclosure may be characterized in terms of their physical properties including, but not limited to, hardness, viscosity, density, elastic moduli, *etc*. The physical quantity of hardness is a measure of the mechanical resistance of a specific material to localized plastic deformation. The term "hardness" as used herein is indicative of the texture of the chewable gel compositions. The hardness value of a material can depend on, for example, how the material is measured, the force applied to the material, temperature, *etc*.

In some embodiments, the hardness of the chewable fill material is measured by a texture analyzer. In some embodiments, the chewable fill materials of the disclosure have a hardness of less than about 800 g. In some embodiments, the chewable fill materials of the disclosure have a hardness of less than about 500 g. In some embodiments, the chewable fill materials of the disclosure have a hardness of less than about 100 g. In some embodiments, the chewable fill materials of the disclosure have a hardness of less than about 50 g. In some embodiments, the chewable fill materials of the disclosure have a hardness of less than about 40 g. In some embodiments, the chewable fill materials of the disclosure have a hardness of less than about 35 g. In some embodiments, the chewable fill materials of the disclosure have a hardness of less than about 30 g. In some embodiments, the chewable fill materials of the disclosure have a hardness of less than about 25 g. In some embodiments, the chewable fill materials of the disclosure have a hardness of less than about 20 g. In some embodiments, the chewable fill materials of the disclosure have a hardness of less than about 15 g. In some embodiments, the chewable fill materials of the disclosure have a hardness in the range of about 0.1 g - 50 g. In some embodiments, the chewable fill materials of the disclosure have a hardness in the range of about 0.1 g - 40 g. In some embodiments, the chewable fill materials of the disclosure have a hardness in the range of about 0.1 g - 35 g. In some embodiments, the chewable fill materials of the disclosure have a hardness in the range of about 0.1 g - 30 g. In some embodiments, the chewable fill materials of the disclosure have a hardness in the range of about 0.1 g - 25 g. In some embodiments, the chewable fill materials of the disclosure have a hardness in the range of about 10 g - 25 g. In some embodiments, the chewable fill materials of the disclosure have a hardness in the range of about 0.1 g - 20 g. In some embodiments, the chewable fill materials of the disclosure have a hardness in the range of about 1 g -20 g. In some embodiments, the chewable fill materials of the disclosure have a hardness in the range of about 5 g - 20 g. In some embodiments, the chewable fill materials of the disclosure have a hardness in the range of about 10 g - 20 g. In some embodiments, the chewable fill materials of the disclosure have a hardness in the range of about 5 g - 15 g. In some embodiments, the chewable fill materials of the disclosure have a hardness in the range of about 0.5 g - 10 g.

Viscosity is a measure of a material's resistance to a change in shape or deformation. In some embodiments, viscosity is measured at a temperature of about 35 °C - 45 °C. In some embodiments, viscosity is measured prior to encapsulation of the fill material by a chewable gel shell. In some embodiments, the chewable fill materials of the disclosure have a viscosity in the range of 0.1 mPa.s to 300 Pa.s when measured at (*e.g.*, heated to) a temperature of about 35 °C - 45 °C prior to encapsulation by the chewable gel shell. In some embodiments, the chewable fill materials of the disclosure have a viscosity in the range of 0.1 mPa.s to 100 Pa.s when measured at (*e.g.*, heated to) a temperature of about 35 °C - 45 °C. In some embodiments, the chewable fill materials of the disclosure have a viscosity in the range of about 100 cP -26,000 cP when measured at a temperature of about 40 °C prior to encapsulation by the chewable gel shell. In some embodiments, the chewable fill materials of the disclosure have a viscosity in the range of about 300 cP -16,000 cP when measured at a temperature of about 40 °C prior to encapsulation by the chewable gel shell. In some embodiments, the chewable fill materials of the disclosure have a viscosity in the range of about 350-15,000 cP, 350-12,500 cP, 400-10,000 cP, 400-9,000 cP, 400-8,000 cP, 500-8,000 cP, 400-7,000 cP, 400-6,000 cP, 400-7,000 cP, 400-6,000 cP, 400-5,000 cP, 400-4,000 cP, 400-3,000 cP, 400-2,000 cP, 400-1,000 cP, or 450-900 cP, when measured at a temperature of about 40 °C prior to encapsulation by the chewable gel shell.

### Gummy-gel forming agent

As used herein, the terms "gummy-gel forming agent", or "gummy-gel forming polymer" refer to any agent that aids in the formation and/or stabilization of the gummy gel and contributes to the chewy texture of the gummy fill material. Gummy-gel forming agents include natural (*e.g.*, food starches, modified starches, collagen peptides), or synthetic polymeric materials that can form gels. Gummy-gel forming agents may be monomers, oligomers, polymers, copolymers or combinations thereof. In some embodiments, the gummy-gel forming agents of the present disclosure include, but are not limited to gelatin, starch (*e.g.*, potato starch, rice starch and corn starch), modified starch (*e.g.*, sodium starch glycolate, modified potato starch, modified rice starch and modified corn starch), pectin, carbomer, poloxamer, agar-agar, carrageenan, or any other gel-forming agents known in the art.

In some embodiments, the gummy-gel forming agent in the chewable fill material is gelatin. Different types of gelatins include, but are not limited to, bovine gelatin, pig gelatin, fish gelatin, skin gelatin, bone gelatin, acid and lime bone gelatin. The term "bovine gelatin" refers to gelatin derived from a bovine source, such as gelatin derived from cow bones, cow skin, cow tendons and/or cow tissues. The term "fish gelatin" refers to a gelatin derived from a fish source such as fish skin and/or fish scales.

In some embodiments, the chewable fill material comprises a total of about 0.1 % - 50% (w/v) of one or more gummy-gel forming agents. In some embodiments, the chewable fill material comprises a total of about 0.1 % - 40% (w/v) of one or more gummy-gel forming agents. In some embodiments, the chewable fill material comprises a total of about 0.1 % - 30% (w/v) of one or more gummy-gel forming agents. In some embodiments, the chewable fill material comprises a total of about 0.1 % - 20% (w/v) of one or more gummy-gel forming agents. In some embodiments, the chewable fill material comprises a total of about 0.1 % - 15% (w/v) of one or more gummy-gel forming agents. In some embodiments, the chewable fill material comprises a total of about 0.1 % - 12% (w/v) of one or more gummy-gel forming agents. In some embodiments, the chewable fill material comprises a total of about 0.1 % - 10% (w/v) of one or more gummy-gel forming agents. In some embodiments, the chewable fill material comprises a total of about 0.5 % - 10% (w/v) of one or more gummy-gel forming agents. In some embodiments, the chewable fill material comprises a total of about 0.1 % - 9% (w/v) of one or more gummy-gel forming agents. In some embodiments, the chewable fill material comprises a total of about 0.1 % - 8% (w/v) of one or more gummy-gel forming agents. In some embodiments, the chewable fill material comprises a total of about 0.1 % - 7% (w/v) of one or more gummy-gel forming agents. In some embodiments, the chewable fill material comprises a total of about 0.1 % - 6.5% (w/v) of one or more gummy-gel forming agents. In some embodiments, the chewable fill material comprises a total of about 0.1 % - 6% (w/v) of one or more gummy-gel forming agents. In some embodiments, the chewable fill material comprises a total of about 0.1 % - 5.5% (w/v) of one or more gummy-gel forming agents. In some embodiments, the chewable fill material comprises a total of about 0.1 % - 5% (w/v) of one or more gummy-gel forming agents. In some embodiments, the chewable fill material comprises a total of about 0.5 % - 5% (w/v) of one or more gummy-gel forming agents. In some embodiments, the chewable fill material comprises a total of about 1 % - 5% (w/v) of one or more gummy-gel forming agents. In some embodiments, the chewable fill material comprises a total of about 2 % - 5% (w/v) of one or more gummy-gel forming agents. In some embodiments, the chewable fill material comprises a total of about 3 % - 6% (w/v) of one or more gummy-gel forming agents. In some embodiments, the chewable fill material comprises a total of about 0.1 % - 4.5% (w/v) of one or more gummy-gel forming agents. In some embodiments, the chewable fill material comprises a total of about 2 % - 25% (w/v) of one or more gummy-gel forming agents. In some embodiments, the chewable fill material comprises a total of about 2 % - 20% (w/v) of one or more gummy-gel forming agents. In some embodiments, the chewable fill material comprises a total of about 2 % - 15% (w/v) of one or more gummy-gel forming agents. In some embodiments, the chewable fill material comprises a total of about 2 % - 13% (w/v) of one or more gummy-gel forming agents. In some embodiments, the chewable fill material comprises a total of about 4 % - 15% (w/v) of one or more gummy-gel forming agents. In some embodiments, the chewable fill material comprises a total of about 4 % - 13% (w/v) of one or more gummy-gel forming agents. In some embodiments, the chewable fill material comprises a total of about 5 % - 15% (w/v) of one or more gummy-gel forming agents. In some embodiments, the chewable fill material comprises a total of about 5 % - 13% (w/v) of one or more gummy-gel forming agents. In some embodiments, the chewable fill material comprises a total of about 7 % - 15% (w/v) of one or more gummy-gel forming agents. In some embodiments, the chewable fill material comprises a total of about 7 % - 13% (w/v) of one or more gummy-gel forming agents. In some embodiments, the chewable fill material comprises a total of about 8 % - 15% (w/v) of one or more gummy-gel forming agents. In some embodiments, the chewable fill material comprises a total of about 8% - 13% (w/v) of one or more gummy-gel forming agents. In some embodiments, the gummy-gel forming agent is gelatin.

In some embodiments, the chewable fill material comprises a total of about 0.1 % - 50% (w/w) of one or more gummy-gel forming agents. In some embodiments, the chewable fill material comprises a total of about 0.1 % - 40% (w/w) of one or more gummy-gel forming agents. In some embodiments, the chewable fill material comprises a total of about 0.1 % - 30% (w/w) of one or more gummy-gel forming agents. In some embodiments, the chewable fill material comprises a total of about 0.1 % - 20% (w/w) of one or more gummy-gel forming agents. In some embodiments, the chewable fill material comprises a total of about 0.1 % - 15% (w/w) of one or more gummy-gel forming agents. In some embodiments, the chewable fill material comprises a total of about 0.1 % - 12% (w/w) of one or more gummy-gel forming agents. In some embodiments, the chewable fill material comprises a total of about 0.1 % - 10% (w/w) of one or more gummy-gel forming agents. In some embodiments, the chewable fill material comprises a total of about 0.5 % - 10% (w/w) of one or more gummy-gel forming agents. In some embodiments, the chewable fill material comprises a total of about 0.1 % - 9% (w/w) of one or more gummy-gel forming agents. In some embodiments, the chewable fill material comprises a total of about 0.1 % - 8% (w/w) of one or more gummy-gel forming agents. In some embodiments, the chewable fill material comprises a total of about 0.1 % - 7% (w/w) of one or more gummy-gel forming agents. In some embodiments, the chewable fill material comprises a total of about 0.1 % - 6.5% (w/w) of one or more gummy-gel forming agents. In some embodiments, the chewable fill material comprises a total of about 0.1 % - 6% (w/w) of one or more gummy-gel forming agents. In some embodiments, the chewable fill material comprises a total of about 0.1 % - 5.5% (w/w) of one or more gummy-gel forming agents. In some embodiments, the chewable fill material comprises a total of about 0.1 % - 5% (w/w) of one or more gummy-gel forming agents. In some embodiments, the chewable fill material comprises a total of about 0.5 % - 5% (w/w) of one or more gummy-gel forming agents. In some embodiments, the chewable fill material comprises a total of about 1 % - 5% (w/w) of one or more gummy-gel forming agents. In some embodiments, the chewable fill material comprises a total of about 2 % - 5% (w/w) of one or more gummy-gel forming agents. In some embodiments, the chewable fill material comprises a total of about 3 % - 6% (w/w) of one or more gummy-gel forming agents. In some embodiments, the chewable fill material comprises a total of about 0.1 % - 4.5% (w/w) of one or more gummy-gel forming agents. In some embodiments, the chewable fill material comprises a total of about 2 % - 25% (w/w) of one or more gummy-gel forming agents. In some embodiments, the chewable fill material comprises a total of about 2 % - 20% (w/w) of one or more gummy-gel forming agents. In some embodiments, the chewable fill material comprises a total of about 2 % - 15% (w/w) of one or more gummy-gel forming agents. In some embodiments, the chewable fill material comprises a total of about 2 % - 13% (w/w) of one or more gummy-gel forming agents. In some embodiments, the chewable fill material comprises a total of about 4 % - 15% (w/w) of one or more gummy-gel forming agents. In some embodiments, the chewable fill material comprises a total of about 4 % - 13% (w/w) of one or more gummy-gel forming agents. In some embodiments, the chewable fill material comprises a total of about 5 % - 15% (w/w) of one or more gummy-gel forming agents. In some embodiments, the chewable fill material comprises a total of about 5 % - 13% (w/w) of one or more gummy-gel forming agents. In some embodiments, the chewable fill material comprises a total of about 7 % - 15% (w/w) of one or more gummy-gel forming agents. In some embodiments, the chewable fill material comprises a total of about 7 % - 13% (w/w) of one or more gummy-gel forming agents. In some embodiments, the chewable fill material comprises a total of about 8 % - 15% (w/w) of one or more gummy-gel forming agents. In some embodiments, the chewable fill material comprises a total of about 8% - 13% (w/w) of one or more gummy-gel forming agents. In some embodiments, the gummy-gel forming agent is gelatin.

As used herein, the term "bloom" or "bloom value" of gelatin refers to a value which is determined by measuring the weight in grams required to move a plunger 0.5 inch in diameter, 4 mm into a 6.67% gelatin gel that has been held for 17 hours at 10 °C. The bloom of gelatin is a measure of the firmness or rigidity of a gelatin gel, with higher numbers indicating a stronger, firmer gel. As used herein, the term "low bloom gelatin" refers to a gelatin having a bloom value between 50-125. As used herein, the term "high bloom gelatin" refers to a gelatin having a bloom value between 250-325. In some embodiments, chewable fill materials of the disclosure comprise gelatin having a bloom value in the range of about 30 - 325. In some embodiments, chewable fill materials of the disclosure comprise gelatin having a bloom value in the range of about 50 - 250.

### Crystallization inhibitor

As used herein, the term "crystallization inhibitor" refers to a substance that is added to prevent or slow down the formation of crystals in the gummy material, *e.g.*, by inhibiting crystallization of sugars. A crystallization inhibitor may also act as a texture modifier of the chewable fill material. In some embodiments, the crystallization inhibitor of the present disclosure includes, but is not limited to, povidone, glucose syrup, corn syrup, fructose syrup, glycerin, hydrogenated starch hydrolysate *etc*. or any combinations thereof. In some embodiments, the crystallization inhibitor comprises glucose syrup. In some embodiments, the crystallization inhibitor comprises povidone. In some embodiments, the crystallization inhibitor comprises povidone and glucose syrup. In some embodiments, the crystallization inhibitor comprises fructose syrup. In some embodiments, the crystallization inhibitor is povidone and fructose syrup. In some embodiments, the crystallization inhibitor comprises glycerin. In some embodiments, the crystallization inhibitor comprises hydrogenated starch hydrolysate.

In some embodiments, the chewable fill material comprises a total of about 0.1 % - 90 % (w/v) of one or more crystallization inhibitors. In some embodiments, the chewable fill material comprises a total of about 0.1 % - 85 % (w/v) of one or more crystallization inhibitors. In some embodiments, the chewable fill material comprises a total of about 0.1 % - 80 % (w/v) of one or more crystallization inhibitors. In some embodiments, the chewable fill material comprises a total of about 1 % - 80 % (w/v) of one or more crystallization inhibitors. In some embodiments, the chewable fill material comprises a total of about 1 % - 70 % (w/v) of one or more crystallization inhibitors. In some embodiments, the chewable fill material comprises a total of about 0.1 % - 60 % (w/v) of one or more crystallization inhibitors. In some embodiments, the chewable fill material comprises a total of about 10 % - 60 % (w/v) of one or more crystallization inhibitors. In some embodiments, the chewable fill material comprises a total of about 20 % - 60 % (w/v) of one or more crystallization inhibitors. In some embodiments, the chewable fill material comprises a total of about 30 % - 75 % (w/v) of one or more crystallization inhibitors. In some embodiments, the chewable fill material comprises a total of about 30 % - 70 % (w/v) of one or more crystallization inhibitors. In some embodiments, the chewable fill material comprises a total of about 30 % - 60 % (w/v) of one or more crystallization inhibitors. In some embodiments, the chewable fill material comprises a total of about 35 % - 60 % (w/v) of one or more crystallization inhibitors. In some embodiments, the chewable fill material comprises a total of about 40 % - 90 % (w/v) of one or more crystallization inhibitors. In some embodiments, the chewable fill material comprises a total of about 40 % - 85 % (w/v) of one or more crystallization inhibitors. In some embodiments, the chewable fill material comprises a total of about 40 % - 80 % (w/v) of one or more crystallization inhibitors. In some embodiments, the chewable fill material comprises a total of about 10 % - 50 % (w/v) of one or more crystallization inhibitors. In some embodiments, the chewable fill material comprises a total of about 20 % - 50 % (w/v) of one or more crystallization inhibitors. In some embodiments, the chewable fill material comprises a total of about 20 % - 45 % (w/v) of one or more crystallization inhibitors. In some embodiments, the chewable fill material comprises a total of about 30 % - 60 % (w/v) of one or more crystallization inhibitors. In some embodiments, the chewable fill material comprises a total of about 40 % - 55 % (w/v) of one or more crystallization inhibitors.

In some embodiments, the chewable fill material comprises a total of about 0.1 % - 90 % (w/w) of one or more crystallization inhibitors. In some embodiments, the chewable fill material comprises a total of about 0.1 % - 85 % (w/w) of one or more crystallization inhibitors. In some embodiments, the chewable fill material comprises a total of about 0.1 % - 80 % (w/w) of one or more crystallization inhibitors. In some embodiments, the chewable fill material comprises a total of about 1 % - 80 % (w/w) of one or more crystallization inhibitors. In some embodiments, the chewable fill material comprises a total of about 1 % - 70 % (w/w) of one or more crystallization inhibitors. In some embodiments, the chewable fill material comprises a total of about 0.1 % - 60 % (w/w) of one or more crystallization inhibitors. In some embodiments, the chewable fill material comprises a total of about 10 % - 60 % (w/w) of one or more crystallization inhibitors. In some embodiments, the chewable fill material comprises a total of about 20 % - 60 % (w/w) of one or more crystallization inhibitors. In some embodiments, the chewable fill material comprises a total of about 30 % - 75 % (w/w) of one or more crystallization inhibitors. In some embodiments, the chewable fill material comprises a total of about 30 % - 70 % (w/w) of one or more crystallization inhibitors. In some embodiments, the chewable fill material comprises a total of about 30 % - 60 % (w/w) of one or more crystallization inhibitors. In some embodiments, the chewable fill material comprises a total of about 35 % - 60 % (w/w) of one or more crystallization inhibitors. In some embodiments, the chewable fill material comprises a total of about 40 % - 90 % (w/w) of one or more crystallization inhibitors. In some embodiments, the chewable fill material comprises a total of about 40 % - 85 % (w/w) of one or more crystallization inhibitors. In some embodiments, the chewable fill material comprises a total of about 40 % - 80 % (w/w) of one or more crystallization inhibitors. In some embodiments, the chewable fill material comprises a total of about 10 % - 50 % (w/w) of one or more crystallization inhibitors. In some embodiments, the chewable fill material comprises a total of about 20 % - 50 % (w/w) of one or more crystallization inhibitors. In some embodiments, the chewable fill material comprises a total of about 20 % - 45 % (w/w) of one or more crystallization inhibitors. In some embodiments, the chewable fill material comprises a total of about 30 % - 60 % (w/w) of one or more crystallization inhibitors. In some embodiments, the chewable fill material comprises a total of about 40 % - 55 % (w/w) of one or more crystallization inhibitors.

In some embodiments, the crystallization inhibitor comprises glucose syrup. In some embodiments, the chewable fill material comprises a total of about 10 % - 80 % (w/v) of glucose syrup. In some embodiments, the chewable fill material comprises a total of about 10 % - 70 % (w/v) of glucose syrup. In some embodiments, the chewable fill material comprises a total of about 10 % - 60 % (w/v) of glucose syrup. In some embodiments, the chewable fill material comprises a total of about 20 % - 60 % (w/v) of glucose syrup. In some embodiments, the chewable fill material comprises a total of about 30 % - 60 % (w/v) of glucose syrup. In some embodiments, the chewable fill material comprises a total of about 40 % - 60 % (w/v) of glucose syrup.

In some embodiments, the crystallization inhibitor comprises glucose syrup. In some embodiments, the chewable fill material comprises a total of about 10 % - 80 % (w/w) of glucose syrup. In some embodiments, the chewable fill material comprises a total of about 10 % - 70 % (w/w) of glucose syrup. In some embodiments, the chewable fill material comprises a total of about 10 % - 60 % (w/w) of glucose syrup. In some embodiments, the chewable fill material comprises a total of about 20 % - 60 % (w/w) of glucose syrup. In some embodiments, the chewable fill material comprises a total of about 30 % - 60 % (w/w) of glucose syrup. In some embodiments, the chewable fill material comprises a total of about 40 % - 60 % (w/w) of glucose syrup.

In some embodiments, the crystallization inhibitor comprises fructose syrup. In some embodiments, the chewable fill material comprises a total of about 10 % - 80 % (w/v) of fructose syrup. In some embodiments, the chewable fill material comprises a total of about 10 % - 70 % (w/v) of fructose syrup. In some embodiments, the chewable fill material comprises a total of about 10 % - 60 % (w/v) of fructose syrup. In some embodiments, the chewable fill material comprises a total of about 20 % - 60 % (w/v) of fructose syrup. In some embodiments, the chewable fill material comprises a total of about 30 % - 60 % (w/v) of fructose syrup. In some embodiments, the chewable fill material comprises a total of about 30 % - 50 % (w/v) of fructose syrup.

In some embodiments, the crystallization inhibitor comprises fructose syrup. In some embodiments, the chewable fill material comprises a total of about 10 % - 80 % (w/w) of fructose syrup. In some embodiments, the chewable fill material comprises a total of about 10 % - 70 % (w/w) of fructose syrup. In some embodiments, the chewable fill material comprises a total of about 10 % - 60 % (w/w) of fructose syrup. In some embodiments, the chewable fill material comprises a total of about 20 % - 60 % (w/w) of fructose syrup. In some embodiments, the chewable fill material comprises a total of about 30 % - 60 % (w/w) of fructose syrup. In some embodiments, the chewable fill material comprises a total of about 30 % - 50 % (w/w) of fructose syrup.

In some embodiments, the crystallization inhibitor comprises povidone and glucose syrup. In some embodiments, the crystallization inhibitor is povidone. In some embodiments, the chewable fill material comprises a total of about 0.1 % - 10 % (w/v) of povidone. In some embodiments, the chewable fill material comprises a total of about 0.1 % - 8 % (w/v) of povidone. In some embodiments, the chewable fill material comprises a total of about 2 % - 8 % (w/v) of povidone. In some embodiments, the chewable fill material comprises a total of about 0.1 % - 6 % (w/v) of povidone. In some embodiments, the chewable fill material comprises a total of about 0.1 % - 5 % (w/v) of povidone. In some embodiments, the chewable fill material comprises a total of about 0.1 % - 4 % (w/v) of povidone. In some embodiments, the chewable fill material comprises a total of about 0.1 % - 3.5 % (w/v) of povidone. In some embodiments, the chewable fill material comprises a total of about 0.1 % - 3 % (w/v) of povidone. In some embodiments, the chewable fill material comprises a total of about 0.5 % - 3 % (w/v) of povidone. In some embodiments, the chewable fill material comprises a total of about 1 % - 3 % (w/v) of povidone.

In some embodiments, the crystallization inhibitor comprises povidone and glucose syrup. In some embodiments, the crystallization inhibitor is povidone. In some embodiments, the chewable fill material comprises a total of about 0.1 % - 10 % (w/w) of povidone. In some embodiments, the chewable fill material comprises a total of about 0.1 % - 8 % (w/w) of povidone. In some embodiments, the chewable fill material comprises a total of about 2 % - 8 % (w/w) of povidone. In some embodiments, the chewable fill material comprises a total of about 0.1 % - 6 % (w/w) of povidone. In some embodiments, the chewable fill material comprises a total of about 0.1 % - 5 % (w/w) of povidone. In some embodiments, the chewable fill material comprises a total of about 0.1 % - 4 % (w/w) of povidone. In some embodiments, the chewable fill material comprises a total of about 0.1 % - 3.5 % (w/w) of povidone. In some embodiments, the chewable fill material comprises a total of about 0.1 % - 3 % (w/w) of povidone. In some embodiments, the chewable fill material comprises a total of about 0.5 % - 3 % (w/w) of povidone. In some embodiments, the chewable fill material comprises a total of about 1 % - 3 % (w/w) of povidone.

In some embodiments, the crystallization inhibitor comprises glycerin. In some embodiments, the chewable fill material comprises a total of about 10 % - 80 % (w/v) of glycerin. In some embodiments, the chewable fill material comprises a total of about 10 % - 70 % (w/v) of glycerin. In some embodiments, the chewable fill material comprises a total of about 10 % - 60 % (w/v) of glycerin. In some embodiments, the chewable fill material comprises a total of about 20 % - 60 % (w/v) of glycerin. In some embodiments, the chewable fill material comprises a total of about 30 % - 60 % (w/v) of glycerin. In some embodiments, the chewable fill material comprises a total of about 40 % - 60 % (w/v) of glycerin.

In some embodiments, the crystallization inhibitor comprises glycerin. In some embodiments, the chewable fill material comprises a total of about 10 % - 80 % (w/w) of glycerin. In some embodiments, the chewable fill material comprises a total of about 10 % - 70 % (w/w) of glycerin. In some embodiments, the chewable fill material comprises a total of about 10 % - 60 % (w/w) of glycerin. In some embodiments, the chewable fill material comprises a total of about 20 % - 60 % (w/w) of glycerin. In some embodiments, the chewable fill material comprises a total of about 30 % - 60 % (w/w) of glycerin. In some embodiments, the chewable fill material comprises a total of about 40 % - 60 % (w/w) of glycerin.

### pH adjusting agent

In some embodiments, the chewable fill material comprises a pH adjusting agent. pH adjusting agents of the disclosure include, but are not limited to, citric acid, malic acid, ascorbic acid, lactic acid, sodium citrate, sodium bicarbonate, *etc*. In some embodiments, the pH adjusting agent is citric acid. In some embodiments, the pH adjusting agent is malic acid.

In some embodiments, the chewable fill material comprises a total of about 0.01 % - 10 % (w/v) of one or more pH adjusting agents. In some embodiments, the chewable fill material comprises a total of about 0.01 % - 5 % (w/v) of one or more pH adjusting agents. In some embodiments, the chewable fill material comprises a total of about 0.1 % - 4 % (w/v) of one or more pH adjusting agents. In some embodiments, the chewable fill material comprises a total of about 0.5 % - 3 % (w/v) of one or more pH adjusting agents. In some embodiments, the chewable fill material comprises a total of about 1 % - 3 % (w/v) of one or more pH adjusting agents. In some embodiments, the chewable fill material comprises a total of about 0.01 % - 3 % (w/v) of one or more pH adjusting agents. In some embodiments, the chewable fill material comprises a total of about 0.01 % - 2 % (w/v) of one or more pH adjusting agents. In some embodiments, the chewable fill material comprises a total of about 0.1 % - 2 % (w/v) of one or more pH adjusting agents. In some embodiments, the chewable fill material comprises a total of about 0.01 % - 1 % (w/v) of one or more pH adjusting agents. In some embodiments, the chewable fill material comprises a total of about 0.1 % - 1 % (w/v) of one or more pH adjusting agents. In some embodiments, the chewable fill material comprises a total of about 0.1 % - 0.9 % (w/v) of one or more pH adjusting agents. In some embodiments, the chewable fill material comprises a total of about 0.1 % - 0.8 % (w/v) of one or more pH adjusting agents. In some embodiments, the chewable fill material comprises a total of about 0.1 % - 0.7 % (w/v) of one or more pH adjusting agents. In some embodiments, the chewable fill material comprises a total of about 0.2 % - 0.8 % (w/v) of one or more pH adjusting agents. In some embodiments, the chewable fill material comprises a total of about 0.2 % - 0.7 % (w/v) of one or more pH adjusting agents.

In some embodiments, the chewable fill material comprises a total of about 0.01 % - 10 % (w/w) of one or more pH adjusting agents. In some embodiments, the chewable fill material comprises a total of about 0.01 % - 5 % (w/w) of one or more pH adjusting agents. In some embodiments, the chewable fill material comprises a total of about 0.1 % - 4 % (w/w) of one or more pH adjusting agents. In some embodiments, the chewable fill material comprises a total of about 0.5 % - 3 % (w/w) of one or more pH adjusting agents. In some embodiments, the chewable fill material comprises a total of about 1 % - 3 % (w/w) of one or more pH adjusting agents. In some embodiments, the chewable fill material comprises a total of about 0.01 % - 3 % (w/w) of one or more pH adjusting agents. In some embodiments, the chewable fill material comprises a total of about 0.01 % - 2 % (w/w) of one or more pH adjusting agents. In some embodiments, the chewable fill material comprises a total of about 0.1 % - 2 % (w/w) of one or more pH adjusting agents. In some embodiments, the chewable fill material comprises a total of about 0.01 % - 1 % (w/w) of one or more pH adjusting agents. In some embodiments, the chewable fill material comprises a total of about 0.1 % - 1 % (w/w) of one or more pH adjusting agents. In some embodiments, the chewable fill material comprises a total of about 0.1 % - 0.9 % (w/w) of one or more pH adjusting agents. In some embodiments, the chewable fill material comprises a total of about 0.1 % - 0.8 % (w/w) of one or more pH adjusting agents. In some embodiments, the chewable fill material comprises a total of about 0.1 % - 0.7 % (w/w) of one or more pH adjusting agents. In some embodiments, the chewable fill material comprises a total of about 0.2 % - 0.8 % (w/w) of one or more pH adjusting agents. In some embodiments, the chewable fill material comprises a total of about 0.2 % - 0.7 % (w/w) of one or more pH adjusting agents.

### Sweeteners

In some embodiments, the chewable fill material comprises one or more sweeteners. Sweeteners of the disclosure include, but are not limited to, sucrose, glycerin, glucose, hydrogenated starch hydrolysate, sucralose, xylitol, artificial sweeteners, natural sweeteners, *etc*., and combinations thereof.

In some embodiments, the chewable fill material comprises a total of about 0.1 % - 80 % (w/v) of one or more sweeteners. In some embodiments, the chewable fill material comprises a total of about 0.1 % - 50 % (w/v) of one or more sweeteners. In some embodiments, the chewable fill material comprises a total of about 0.1 % - 40 % (w/v) of one or more sweeteners. In some embodiments, the chewable fill material comprises a total of about 0.1 % - 35 % (w/v) of one or more sweeteners. In some embodiments, the chewable fill material comprises a total of about 0.1 % - 30 % (w/v) of one or more sweeteners. In some embodiments, the chewable fill material comprises a total of about 5 % - 30 % (w/v) of one or more sweeteners. In some embodiments, the chewable fill material comprises a total of about 0.1 % - 25 % (w/v) of one or more sweeteners. In some embodiments, the chewable fill material comprises a total of about 4 % - 34 % (w/v) of one or more sweeteners. In some embodiments, the chewable fill material comprises a total of about 5 % - 33 % (w/v) of one or more sweeteners. In some embodiments, the chewable fill material comprises a total of about 5 % - 32 % (w/v) of one or more sweeteners. In some embodiments, the chewable fill material comprises a total of about 6 % - 31 % (w/v) of one or more sweeteners. In some embodiments, the chewable fill material comprises a total of about 5 % - 20 % (w/v) of one or more sweeteners.

In some embodiments, the chewable fill material comprises a total of about 0.1 % - 80 % (w/w) of one or more sweeteners. In some embodiments, the chewable fill material comprises a total of about 0.1 % - 50 % (w/w) of one or more sweeteners. In some embodiments, the chewable fill material comprises a total of about 0.1 % - 40 % (w/w) of one or more sweeteners. In some embodiments, the chewable fill material comprises a total of about 0.1 % - 35 % (w/w) of one or more sweeteners. In some embodiments, the chewable fill material comprises a total of about 0.1 % - 30 % (w/w) of one or more sweeteners. In some embodiments, the chewable fill material comprises a total of about 5 % - 30 % (w/w) of one or more sweeteners. In some embodiments, the chewable fill material comprises a total of about 0.1 % - 25 % (w/w) of one or more sweeteners. In some embodiments, the chewable fill material comprises a total of about 4 % - 34 % (w/w) of one or more sweeteners. In some embodiments, the chewable fill material comprises a total of about 5 % - 33 % (w/w) of one or more sweeteners. In some embodiments, the chewable fill material comprises a total of about 5 % - 32 % (w/w) of one or more sweeteners. In some embodiments, the chewable fill material comprises a total of about 6 % - 31 % (w/w) of one or more sweeteners. In some embodiments, the chewable fill material comprises a total of about 5 % - 20 % (w/w) of one or more sweeteners.

In some embodiments, the chewable fill material comprises hydrogenated starch hydrolysate. Hydrogenated starch hydrolysate refers to a mixture of sugar alcohols, including maltitol, sorbitol, *etc*., created by hydrogenating starch. In some embodiments, the chewable fill material comprises about 1 % - 35 % (w/v) hydrogenated starch hydrolysate. In some embodiments, the chewable fill material comprises about 1 % - 30 % (w/v) hydrogenated starch hydrolysate. In some embodiments, the chewable fill material comprises about 5 % - 30 % (w/v) hydrogenated starch hydrolysate. In some embodiments, the chewable fill material comprises about 1 % - 25 % (w/v) hydrogenated starch hydrolysate. In some embodiments, the chewable fill material comprises about 5 % - 25 % (w/v) hydrogenated starch hydrolysate. In some embodiments, the chewable fill material comprises about 10 % - 30 % (w/v) hydrogenated starch hydrolysate. In some embodiments, the chewable fill material comprises about 10 % - 25 % (w/v) hydrogenated starch hydrolysate. In some embodiments, the chewable fill material comprises about 15 % - 30 % (w/v) hydrogenated starch hydrolysate. In some embodiments, the chewable fill material comprises about 15 % - 25 % (w/v) hydrogenated starch hydrolysate. In some embodiments, the chewable fill material comprises about 2 % -34 % (w/v) hydrogenated starch hydrolysate. In some embodiments, the chewable fill material comprises about 3% - 33 % (w/v) hydrogenated starch hydrolysate. In some embodiments, the chewable fill material comprises about 4 % -32 % (w/v) hydrogenated starch hydrolysate. In some embodiments, the chewable fill material comprises about 5 % - 30 % (w/v) hydrogenated starch hydrolysate. In some embodiments, the chewable fill material comprises about 5 % - 29 % (w/v) hydrogenated starch hydrolysate.

In some embodiments, the chewable fill material comprises about 1 % - 35 % (w/w) hydrogenated starch hydrolysate. In some embodiments, the chewable fill material comprises about 1 % - 30 % (w/w) hydrogenated starch hydrolysate. In some embodiments, the chewable fill material comprises about 5 % - 30 % (w/w) hydrogenated starch hydrolysate. In some embodiments, the chewable fill material comprises about 1 % - 25 % (w/w) hydrogenated starch hydrolysate. In some embodiments, the chewable fill material comprises about 5 % - 25 % (w/w) hydrogenated starch hydrolysate. In some embodiments, the chewable fill material comprises about 10 % - 30 % (w/w) hydrogenated starch hydrolysate. In some embodiments, the chewable fill material comprises about 10 % - 25 % (w/w) hydrogenated starch hydrolysate. In some embodiments, the chewable fill material comprises about 15 % - 30 % (w/w) hydrogenated starch hydrolysate. In some embodiments, the chewable fill material comprises about 15 % - 25 % (w/w) hydrogenated starch hydrolysate. In some embodiments, the chewable fill material comprises about 2 % -34 % (w/w) hydrogenated starch hydrolysate. In some embodiments, the chewable fill material comprises about 3% - 33 % (w/w) hydrogenated starch hydrolysate. In some embodiments, the chewable fill material comprises about 4 % -32 % (w/w) hydrogenated starch hydrolysate. In some embodiments, the chewable fill material comprises about 5 % - 30 % (w/w) hydrogenated starch hydrolysate. In some embodiments, the chewable fill material comprises about 5 % - 29 % (w/w) hydrogenated starch hydrolysate.

In some embodiments, the chewable fill material comprises glycerin. In some embodiments, the chewable fill material comprises about 0.1 % - 20 % (w/v) glycerin. In some embodiments, the chewable fill material comprises about 0.1 % - 10 % glycerin. In some embodiments, the chewable fill material comprises about 1 % - 10 % (w/v) glycerin. In some embodiments, the chewable fill material comprises about 5 % - 10 % (w/v) glycerin.

In some embodiments, the chewable fill material comprises about 0.1 % - 20 % (w/w) glycerin. In some embodiments, the chewable fill material comprises about 0.1 % - 10 % (w/w) glycerin. In some embodiments, the chewable fill material comprises about 1 % - 10 % (w/w) glycerin. In some embodiments, the chewable fill material comprises about 5 % - 10 % (w/w) glycerin.

In some embodiments, the chewable fill material comprises xylitol. In some embodiments, the chewable fill material comprises about 0.1 % - 30 % (w/v) xylitol. In some embodiments, the chewable fill material comprises about 0.1 % - 25 % (w/v) xylitol. In some embodiments, the chewable fill material comprises about 1 % - 25 % (w/v) xylitol. In some embodiments, the chewable fill material comprises about 0.1 % - 20 % (w/v) xylitol. In some embodiments, the chewable fill material comprises about 1 % - 20 % (w/v) xylitol. In some embodiments, the chewable fill material comprises about 5 % - 25 % (w/v) xylitol. In some embodiments, the chewable fill material comprises about 5 % - 20 % (w/v) xylitol. In some embodiments, the chewable fill material comprises about 10 % - 20 % (w/v) xylitol.

In some embodiments, the chewable fill material comprises about 0.1 % - 30 % (w/w) xylitol. In some embodiments, the chewable fill material comprises about 0.1 % - 25 % (w/w) xylitol. In some embodiments, the chewable fill material comprises about 1 % - 25 % (w/w) xylitol. In some embodiments, the chewable fill material comprises about 0.1 % - 20 % (w/w) xylitol. In some embodiments, the chewable fill material comprises about 1 % - 20 % (w/w) xylitol. In some embodiments, the chewable fill material comprises about 5 % - 25 % (w/w) xylitol. In some embodiments, the chewable fill material comprises about 5 % - 20 % (w/w) xylitol. In some embodiments, the chewable fill material comprises about 10 % - 20 % (w/w) xylitol.

### Active agent

As used herein, the terms "active agent", "active ingredient" or "active pharmaceutical ingredient" includes therapeutic, nutritional or diagnostic agents. Active agents include, but are not limited to, medicaments (*e.g.,* pharmaceutical or nutraceutical compounds), vitamins, minerals, essential oils, herbal extracts, *etc*., and combinations thereof. Exemplary active agents include Dextromethorphan HBr, Doxylamine Succinate, Diphenhydramine HCl, ubiquinol, creatine, lutein, zeaxanthin, ibuprofen, guaifenesin, acetaminophen, triprolidine HCl, CoQ10, or combinations thereof. In some embodiments, the active ingredient comprises any common dietary supplement known in the art such.

Active agents may be present in therapeutically effective amounts or prophylactically effective amounts in the chewable fill materials of the disclosure. Those skilled in the art will recognize, however, that a pharmaceutical composition and/or nutraceutical composition may contain more than a therapeutically effective amount, such as in bulk compositions, or less than a therapeutically effective amount, that is, individual unit doses designed for multiple administration to achieve a therapeutically effective amount. Typically, the actual amount will depend upon the formulation itself, the route of administration, the frequency of dosing, and so forth.

Active agents of the present disclosure may be administered in a manner appropriate to the condition, disease, and/or disorder to be treated (or prevented). The quantity and frequency of administration will be determined by such factors as the condition of the patient, and the type and severity of the patient's condition, disease, and/or disorder, although appropriate dosages may be determined by clinical trials.

In some embodiments, the chewable fill material comprises a total of about 0.01 % - 50 % (w/v) active agent. In some embodiments, the chewable fill material comprises a total of about 0.01 % - 20 % (w/v) active agent. In some embodiments, the chewable fill material comprises about 0.01 % - 15 % (w/v) active agent. In some embodiments, the chewable fill material comprises about 0.1 % - 15 % (w/v) active agent. In some embodiments, the chewable fill material comprises about 0.5 % - 15 % (w/v) active agent. In some embodiments, the chewable fill material comprises about 0.1 % - 10 % (w/v) active agent. In some embodiments, the chewable fill material comprises about 0.2 % - 8 % (w/v) active agent

In some embodiments, the chewable fill material comprises a total of about 0.01 % - 50 % (w/w) active agent. In some embodiments, the chewable fill material comprises a total of about 0.01 % - 20 % (w/w) active agent. In some embodiments, the chewable fill material comprises about 0.01 % - 15 % (w/w) active agent. In some embodiments, the chewable fill material comprises about 0.1 % - 15 % (w/w) active agent. In some embodiments, the chewable fill material comprises about 0.5 % - 15 % (w/w) active agent. In some embodiments, the chewable fill material comprises about 0.1 % - 10 % (w/w) active agent. In some embodiments, the chewable fill material comprises about 0.2 % - 8 % (w/w) active agent.

In some embodiments, the chewable fill material comprises one or more active agents. In some embodiments, the chewable fill material comprises one active agent. In some embodiments, the chewable fill material comprises two active agents. In some embodiments, the chewable fill material comprises three or more active agents.

### Additives

As used herein, the term "additive" includes, but is not limited to, flavoring agents (*e.g.*, vanillin, lemon oil, citric acid, natural flavors, artificial flavors, essential oils, dairy flavors, herbal extracts, *etc.*), colors (*e.g.*, natural and artificial colorants), or other taste-masking components. In some embodiments, the additive is a flavoring agent.

In some embodiments, the chewable fill material comprises a total of about 0.01 % - 30 % (w/v) additive. In some embodiments, the chewable fill material comprises a total of about 0.01 % - 10 % (w/v) additive. In some embodiments, the chewable fill material comprises a total of about 0.01 % - 8 % (w/v) additive. In some embodiments, the chewable fill material comprises a total of about 0.01 % - 5 % (w/v) additive. In some embodiments, the chewable fill material comprises a total of about 0.1 % - 5 % (w/v) additive. In some embodiments, the chewable fill material comprises a total of about 0.1 % - 2 % (w/v) additive. In some embodiments, the chewable fill material comprises a total of about 0.5 % - 2 % (w/v) additive. In some embodiments, the chewable fill material comprises a total of about 1.0 % - 2 % (w/v) additive.

In some embodiments, the chewable fill material comprises a total of about 0.01 % - 30 % (w/w) additive. In some embodiments, the chewable fill material comprises a total of about 0.01 % - 10 % (w/w) additive. In some embodiments, the chewable fill material comprises a total of about 0.01 % - 8 % (w/w) additive. In some embodiments, the chewable fill material comprises a total of about 0.01 % - 5 % (w/w) additive. In some embodiments, the chewable fill material comprises a total of about 0.1 % - 5 % (w/w) additive. In some embodiments, the chewable fill material comprises a total of about 0.1 % - 2 % (w/w) additive. In some embodiments, the chewable fill material comprises a total of about 0.5 % - 2 % (w/w) additive. In some embodiments, the chewable fill material comprises a total of about 1.0 % - 2 % (w/w) additive.

### Solvent

In some embodiments, the chewable fill material comprises one or more solvents. Solvents include, but are not limited to, water, lipid, oil, propylene glycol, polyethylene glycol (PEG), medium-chain triglycerides (MCT), *etc*., and combinations thereof. In some embodiments, the solvent comprises propylene glycol.

In some embodiments, the chewable fill material comprises a total of about 0.1 % - 10 % (w/v) propylene glycol. In some embodiments, the chewable fill material comprises a total of about 0.1 % - 5 % (w/v) propylene glycol. In some embodiments, the chewable fill material comprises a total of about 0.1 % - 3% (w/v) propylene glycol. In some embodiments, the chewable fill material comprises a total of about 0.1 % - 2 % (w/v) propylene glycol. In some embodiments, the chewable fill material comprises a total of about 0.5 % - 2 % (w/v) propylene glycol.

In some embodiments, the chewable fill material comprises a total of about 0.1 % - 10 % (w/w) propylene glycol. In some embodiments, the chewable fill material comprises a total of about 0.1 % - 5 % (w/w) propylene glycol. In some embodiments, the chewable fill material comprises a total of about 0.1 % - 3% (w/w) propylene glycol. In some embodiments, the chewable fill material comprises a total of about 0.1 % - 2 % (w/w) propylene glycol. In some embodiments, the chewable fill material comprises a total of about 0.5 % - 2 % (w/w) propylene glycol.

### Lipids

In some embodiments, the chewable fill material comprises one or more lipids. The inclusion of lipids in the gummy-gel fill material may improve the mouth-feel of the gummy-gel. In addition, the lipid may act as a secondary plasticizing stabilizer and/or as a moisture-barrier regulator to reduce the rate of water loss from a gummy-gel of the disclosure. In some embodiments, addition of a lipid (*e.g.*, MCT oil) reduces water loss from the chewable fill material by at least 1.1-fold, at least 1.2-fold, at least 1.5-fold, at least 2-fold, at least 5-fold, at least 10-fold, at least 50-fold, at least 100-fold compared to a fill material which does not comprise the lipid.

Exemplary lipids include, but are not limited to, MCT oil, commonly used fats or oils (*e.g.*, olive oil, sunflower oil, fish oil, krill oil, flaxseed oil, hemp oil, chia oil, evening primrose oil, borage oil, black cumin oil, avocado oil, etc.), eicosapentaenoic acid (EPA), docosahexaenoic acid (DHA), and wax (*e.g.*, beeswax, carnauba wax, rice bran, candelilla, etc.). In some embodiments, the chewable fill material comprises MCT oil.

In some embodiments, the chewable fill material comprises a total of about 0.1 % - 20 % (w/v) lipid. In some embodiments, the chewable fill material comprises a total of about 0.1 % - 15 % (w/v) lipid. In some embodiments, the chewable fill material comprises a total of about 1 % - 15 % (w/v) lipid. In some embodiments, the chewable fill material comprises a total of about 5 % - 15 % (w/v) lipid. In some embodiments, the chewable fill material comprises a total of about 5% - 12% (w/v) lipid. In some embodiments, the chewable fill material comprises a total of about 5% - 10 % (w/v) lipid.

n some embodiments, the chewable fill material comprises a total of about 0.1 % - 20 % (w/w) lipid. In some embodiments, the chewable fill material comprises a total of about 0.1 % - 15 % (w/w) lipid. In some embodiments, the chewable fill material comprises a total of about 1 % - 15 % (w/w) lipid. In some embodiments, the chewable fill material comprises a total of about 5 % - 15 % (w/w) lipid. In some embodiments, the chewable fill material comprises a total of about 5% - 12% (w/w) lipid. In some embodiments, the chewable fill material comprises a total of about 5% - 10 % (w/w) lipid.

In some embodiments, the chewable fill material comprises a total of about 0.1 % - 20 % (w/v) MCT. In some embodiments, the chewable fill material comprises a total of about 0.1 % - 12 % (w/v) MCT. In some embodiments, the chewable fill material comprises a total of about 1 % - 10% (w/v) MCT. In some embodiments, the chewable fill material comprises a total of about 2 % - 10 % (w/v) MCT. In some embodiments, the chewable fill material comprises a total of about 5 % - 10 % (w/v) MCT. In some embodiments, the chewable fill material comprises a total of about 0.1 % - 20 % (w/v) MCT. In some embodiments, the chewable fill material comprises a total of about 0.1 % - 15 % (w/v) MCT. In some embodiments, the chewable fill material comprises a total of about 1 % - 15% (w/v) MCT. In some embodiments, the chewable fill material comprises a total of about 2 % - 15 % (w/v) MCT. In some embodiments, the chewable fill material comprises a total of about 5 % - 12 % (w/v) MCT.

In some embodiments, the chewable fill material comprises a total of about 0.1 % - 20 % (w/w) MCT. In some embodiments, the chewable fill material comprises a total of about 0.1 % - 12 % (w/w) MCT. In some embodiments, the chewable fill material comprises a total of about 1 % - 10% (w/w) MCT. In some embodiments, the chewable fill material comprises a total of about 2 % - 10 % (w/w) MCT. In some embodiments, the chewable fill material comprises a total of about 5 % - 10 % (w/w) MCT. In some embodiments, the chewable fill material comprises a total of about 0.1 % - 20 % (w/w) MCT. In some embodiments, the chewable fill material comprises a total of about 0.1 % - 15 % (w/w) MCT. In some embodiments, the chewable fill material comprises a total of about 1 % - 15% (w/w) MCT. In some embodiments, the chewable fill material comprises a total of about 2 % - 15 % (w/w) MCT. In some embodiments, the chewable fill material comprises a total of about 5 % - 12 % (w/w) MCT.

### Surfactants or Emulsifiers

In some embodiments, the chewable fill material comprises one or more surfactants. In some embodiments, the chewable fill material comprises one or more emulsifiers. Surfactants or emulsifiers include, but are not limited to, polysorbate, medium chain macrogol-8 glycerides, lecithin, *etc*.

### Gel shell

The compositions of the present disclosure comprise a chewable gel shell encapsulating the chewable fill material, *e.g.*, softgel capsules. The chewable gel shell comprises at least one gelling agent. In some embodiments, the chewable gel shell further comprises one or more of plasticizers, anti-tacking agents, softening agents, sweeteners, flavoring agents, colorants, preservatives, water and combinations thereof.

As used herein, the terms "gelling agent", "gel-forming polymer", or "gel-forming composition" refer to any agent that aids in the formation of a gel and/or stabilization of a gel, or to any agent that retains moisture. Gelling agents include natural (*e.g.*, food starches, modified starches, collagen peptides), or synthetic polymeric materials that can form gels. Gelling agents may be monomers, oligomers, polymers, copolymers or combinations thereof. In some embodiments, the gelling agents of the present disclosure include, but are not limited to gelatin, starch, modified starch, pectin, carbomer, poloxamer, agar-agar, carrageenan, or any other gelling agents known in the art.

Examples of gel forming agents used in the chewable gel shell include, but are not limited to, gelatins (*e.g.*, bovine gelatin, pig gelatin, fish gelatin, skin gelatin, bone gelatin, acid and lime bone gelatin *etc*.), food starches (*e.g.*, potato starch, rice starch and corn starch), modified starches (*e.g.*, sodium starch glycolate, modified potato starch, modified rice starch and modified corn starch), and bovine collagen peptides.

In some embodiments, chewable gel shells of the disclosure comprise gelatin. In some embodiments, the chewable gel shell comprises from about 10% - 50% (w/v) of a low bloom gelatin. In some embodiments, chewable gel shells of the disclosure comprise gelatin. In some embodiments, the chewable gel shell comprises from about 10% - 40% (w/v) of a low bloom gelatin. In some embodiments, the chewable gel shell comprises from about 10 - 35% (w/v) of a low bloom gelatin. In some embodiments, the chewable gel shell comprises from about 15% - 35% (w/v) of a low bloom gelatin. In some embodiments, the chewable gel shell comprises from about 10% - 25% (w/v) of a low bloom gelatin. In some embodiments, the chewable gel shell comprises from about 15% - 25% (w/v) of a low bloom gelatin. In some embodiments, the chewable gel shell comprises from about 1% - 20% (w/v) of a high bloom gelatin. In some embodiments, the chewable gel shell comprises from about 3% - 20% (w/v) of a high bloom gelatin. In some embodiments, the chewable gel shell comprises from about 1% - 15% (w/v) of a high bloom gelatin. In some embodiments, the chewable gel shell comprises from about 3% - 15% (w/v) of a high bloom gelatin. In some embodiments, the chewable gel shell comprises from about 1% - 10% (w/v) of a high bloom gelatin. In some embodiments, the chewable gel shell comprises from about 3% - 10% (w/v) of a high bloom gelatin.

In some embodiments, the weight ratio of low bloom gelatin: high bloom gelatin in the chewable gel shell is from about 1.0 to about 3.5. In some embodiments, the weight ratio of low bloom gelatin: high bloom gelatin is from about 1.5 to about 3.2, or from about 1.7 to about 3.1. In some embodiments, the amount of low bloom gelatin, prior to drying, ranges from about 16.5% - 25% (w/v), or from about 18% - 20% (w/v). In some embodiments, the amount of high bloom gelatin, prior to drying, ranges from about 4% - 14% (w/v), or from about 5% - 10%, (w/v) or from about 6% - 7% (w/v).

In some embodiments, chewable gel shells of the disclosure comprise a starch (*e.g.*, plant starch and/or modified starch). In some embodiments, the chewable gel shell comprises from about 8% - 35% (w/v) of starch. In some embodiments, the chewable gel shell comprises from about 8% - 30% (w/v) of starch. In some embodiments, the chewable gel shell comprises from about 8% -25% (w/v) of starch. In some embodiments, the chewable gel shell comprises from about 8% - 20% (w/v) of starch. In some embodiments, the chewable gel shell comprises from about 10% - 20% (w/v) of starch.

Plasticizers may be added to the chewable gel shell to improve elasticity and durability, and to minimize brittleness and cracking. Examples of plasticizers include, but are not limited to, glycerin, sorbitol, sorbitan, maltitol, polyethylene glycols (PEG), *etc*. In some embodiments, chewable gel shells of the disclosure comprises about 20% - 30 % (w/v) of one or more plasticizers. In some embodiments, chewable gel shells of the disclosure comprises about 25% - 30 % (w/v) of one or more plasticizers.

The chewable gel shells of the disclosure may further comprise one or more anti-tacking and softening agents. Examples of anti-tacking and softening agents include, but are not limited to, lecithins (*e.g.*, sunflower lecithin, soy lecithin *etc*.), hydrogenated vegetable oil, beeswax, coconut oil, palm oil and combinations thereof. In some embodiments, chewable gel shells of the disclosure comprises about 0.1 % - 10 % (w/v) of one or more anti-tacking and softening agents. In some embodiments, chewable gel shells of the disclosure comprises about 0.1 % - 5 % (w/v) of one or more anti-tacking and softening agents.

The chewable gel shells of the disclosure may further comprise one or more flavoring agents. Examples of flavoring agents include, but are not limited to, vanillin, lemon oil, citric acid, and natural and artificial flavors, and combinations thereof. In some embodiments, chewable gel shells of the disclosure comprises about 0.1 % - 5 % (w/v) of one or more flavoring agents.

The chewable gel shells of the disclosure may further comprise one or more colorants and/or one or more preservatives (*e.g.*, citric acid, potassium sorbate, rosemary extract, alpha tocopherol, *etc*.).

### Methods Of Making Chewable Gummy-Gels

The inventors have surprisingly discovered that chewable gummy fill materials of the disclosure can be encapsulated in a chewable gel shell using a softgel rotary die process. The formulations of the gummy compositions disclosed herein have rheological profiles which are advantageous for processability of the gummy fill material in a rotary die process. As a result, the chewable fill material is encapsulated at much lower temperatures compared to conventional gummy manufacturing processes. The rotary die process disclosed herein is thus suitable for encapsulation of fill materials containing active compounds which are susceptible to thermal degradation. Moreover, encapsulation protects the active compound from exposure to light, atmosphere, oxidation, etc., thereby improving the long-term stability of the active compound.

Accordingly, in one aspect, the present disclosure provides a method of making a chewable gummy-gel comprising the steps of preparing a chewable fill material mixture comprising one or more gummy-gel forming agents and one or more crystallization inhibitors; incubating the mixture at a fixed temperature for a time period sufficient to achieve a uniform mixture; and encapsulating the uniform mixture by a chewable gel shell using a rotary die process.

The chewable fill material mixture is prepared by mixing components (*e.g.*, gummy-gel forming agents, crystallizations inhibitors, pH adjusting agents, active agents, sweeteners, flavors, water and combinations thereof) using appropriate mixing equipment. As used herein, the term "mixture" may refer to a mixture of all the components used in the chewable fill material, or a mixture of a subset of the components used in the chewable fill material (*e.g.*, gel-forming polymer and water, crystallization inhibitor, sweetener, pH adjusting agent and water). In some embodiments, the mixture is incubated at a temperature ranging from about 20-80 °C. In some embodiments, the mixture is incubated at a temperature ranging from about 20-75 °C. In some embodiments, the mixture is incubated at a temperature ranging from about 25-80 °C. In some embodiments, the mixture is incubated at a temperature ranging from about 25-75 °C. In some embodiments, the mixture is incubated at a temperature ranging from about 25-60 °C. In some embodiments, the mixture is incubated at a temperature ranging from about 35-70 °C. In some embodiments, the mixture is incubated at a temperature ranging from about 40-70 °C. In some embodiments, the mixture is incubated at a temperature ranging from about 45-70 °C. In some embodiments, the mixture is incubated at a temperature ranging from about 50-70 °C. In some embodiments, the mixture is incubated at a temperature ranging from about 50-65 °C. In some embodiments, the mixture is incubated at a temperature ranging from about 55-65°C.

In some embodiments, the mixture is incubated for a time period of about 0.5 - 48 hours until a uniform mixture is achieved. In some embodiments, the mixture is incubated for a time period of about 0.5 - 40 hours until a uniform mixture is achieved. In some embodiments, the mixture is incubated for a time period of about 0.5 - 36 hours until a uniform mixture is achieved. In some embodiments, the mixture is incubated for a time period of about 2 - 36 hours until a uniform mixture is achieved. In some embodiments, the mixture is incubated for a time period of about 10 - 36 hours until a uniform mixture is achieved.

In some embodiments, the chewable fill material mixture of the disclosure has a viscosity in the range of about 100 cP -26,000 cP when measured at a temperature of about 40 °C. In some embodiments, the fill material mixture has a viscosity of about 300-16,000 cP at a temperature of about 40 °C. In some embodiments, the fill material mixture has a viscosity of about 350-15,000 cP at a temperature of about 40 °C. In some embodiments, the fill material mixture has a viscosity of about 350-12,500 cP at a temperature of about 40 °C In some embodiments, the fill material mixture has a viscosity of about 400-10,000 cP at a temperature of about 40 °C. In some embodiments, the fill material mixture has a viscosity of about 400-9,000 cP at a temperature of about 40 °C. In some embodiments, the fill material mixture has a viscosity of about 400-8,000 cP at a temperature of about 40 °C. In some embodiments, the fill material mixture has a viscosity of about 500-8,000 cP at a temperature of about 40 °C. In some embodiments, the fill material mixture has a viscosity of about 400-7,000 cP at a temperature of about 40 °C. In some embodiments, the fill material mixture has a viscosity of about 400-6,000 cP at a temperature of about 40 °C. In some embodiments, the fill material mixture has a viscosity of about 400-5,000 cP at a temperature of about 40 °C. In some embodiments, the fill material mixture has a viscosity of about 400-4,000 cP at a temperature of about 40 °C. In some embodiments, the fill material mixture has a viscosity of about 400-3,000 cP at a temperature of about 40 °C. In some embodiments, the fill material mixture has a viscosity of about 400-2,000 cP at a temperature of about 40 °C. In some embodiments, the fill material mixture has a viscosity of about 400-1,000 cP at a temperature of about 40 °C. In some embodiments, the fill material mixture has a viscosity of about 400-900 cP at a temperature of about 40 °C. In some embodiments, the fill material mixture has a viscosity of about 450-900 cP at a temperature of about 40 °C.

The uniform mixture of the fill material is encapsulated by a chewable gel shell using a rotary die process. In a typical rotary die process, a molten mass of a gel shell formulation is fed from a reservoir onto drums to form two spaced sheets or ribbons in a semi-molten state. These ribbons are fed around rollers and brought together at a convergent angle into the nip of a pair of roller dies that include opposed die cavities. The chewable fill material containing the active agent or other agent to be encapsulated is fed into the wedge-shaped joiner of the ribbons.

The gel shell ribbons can be continuously conveyed between the dies, with the active agent being trapped between the sheets inside the die cavities. The sheets can then be pressed together, and severed around each die so that opposed edges of the sheets flow together to form a continuous sheath around the entrapped active agent. Alternatively, the active agent can be incorporated into the gel shell sheaths. The part of the sheet that is severed from the segments forming the capsules can be collected for recycling. The very soft capsules can then be dried to increase the integrity of the sheath, and packaged for later distribution and consumption.

In some embodiments, chewable gel compositions of the disclosure improve the stability of the active compound compared to a control. The stability of the active compound may be measured by the amount of degradation or oxidation products of the active compound. Various methods for detecting and quantifying the amount of degradation or oxidation products are known in the art and include, but are not limited to, high performance liquid chromatography (HPLC), gas chromatography, mass spectrometry, spectroscopy, surface plasmon resonance, nuclear magnetic resonance, etc. In some embodiments, chewable gel compositions of the disclosure decrease the degradation of the active compound compared to a control. In some embodiments, chewable gel compositions of the disclosure decrease the degradation of the active compound by at least 1.1-fold, at least 1.2-fold, at least 1.5-fold, at least 2-fold, at least 5-fold, at least 10-fold, at least 50-fold, at least 100-fold compared to a control. In some embodiments, active compounds in chewable gel compositions of the disclosure show minimal (<10%) degradation for at least 1 day, at least 2 days, at least 5 days, at least 1 week, at least 2 weeks, at least 3 weeks, at least 1 month, at least 2 months, at least 3 months, at least 4 months, at least 5 months, at least 6 months, or at least 1 year. In some embodiments, the control is a chewable fill material not encapsulated by a chewable gel shell. In some embodiments, the control is a chewable fill material prepared at a higher temperature.

### EXAMPLES

The chewable fill material was prepared by mixing all components using appropriate mixing equipment, and incubating the mixture at a temperature ranging from 25-70 °C for a time period until a uniform mixture is achieved. The resulting uniform mixture of fill material was encapsulated into a chewable gel shell using a rotary die process.

Exemplary formulations of chewable fill material are provided in the tables below.

### Example 1

| **Components** | **Content (% W/V)** | **Content (% W/W)** |
|---|---|---|
| Glucose Syrup | 53 | 50.5 |
| Hydrogenated Starch Hydrolysate | 24 | 22.9 |
| Propylene Glycol | 1.6 | 1.5 |
| Povidone | 2.4 | 2.3 |
| Dextromethorphan HBr | 0.4 | 0.4 |
| Citric Acid | 0.6 | 0.6 |
| Sweetener | 0.5 | 0.5 |
| Flavor | 1.2 | 1.1 |
| Gelatin | 5.0 | 4.8 |
| Water | quantum satis (QS) | 15.5 |

### Example 2

| **Components** | **Content (% W/V)** | **Content (% W/W)** |
|---|---|---|
| Glucose Syrup | 53 | 49.8 |
| Xylitol | 16 | 15.0 |
| Glycerin | 8 | 7.5 |
| Propylene Glycol | 1.6 | 1.5 |
| Povidone | 2.4 | 2.3 |
| Dextromethorphan HBr | 0.4 | 0.4 |
| Doxylamine Succinate | 0.3 | 0.3 |
| Citric Acid | 0.6 | 0.6 |
| Sweetener | 0.5 | 0.5 |
| Flavor | 1.6 | 1.5 |
| Gelatin | 4.0 | 3.8 |
| Water | quantum satis (QS) | 17.0 |

### Example 3

| **Components** | **Content (% W/V)** | **Content (% W/W)** |
|---|---|---|
| Glucose Syrup | 53 | 49.5 |
| Hydrogenated Starch Hydrolysate | 24 | 22.4 |
| Propylene Glycol | 1.6 | 1.5 |
| Povidone | 2.4 | 2.2 |
| Diphenhydramine HCl | 1.0 | 0.9 |
| Citric Acid | 0.6 | 0.6 |
| Sweetener | 0.5 | 0.5 |
| Flavor | 1.2 | 1.1 |
| Gelatin | 4.7 | 4.4 |
| Water | quantum satis (QS) | 16.8 |

### Example 4

| **Components** | **Content (% W/V)** | **Content (% W/W)** |
|---|---|---|
| Fructose Syrup | 43 | 41.3 |
| Xylitol | 15 | 14.4 |
| Glycerin | 7.4 | 7.1 |
| Citric Acid | 0.6 | 0.6 |
| CoQ10 | 7.4 | 7.1 |
| MCT | 7.4 | 7.1 |
| Flavor | 1.5 | 1.4 |
| Gelatin | 3.4 | 3.3 |
| Water | quantum satis (QS) | 17.6 |

### Example 5: Evaluation of the viscosity of chewable fill material mixtures

The viscosity of multiple chewable fill material mixtures were evaluated. Fill material mixtures corresponding to those disclosed in International Publication No. WO 2003/090726 were prepared as shown in Formulas 1-4 in Table 1. Gelatin hydrolysate in these mixtures was replaced by a crystallization inhibitor (glucose syrup), as shown in Formulas 5-8 in Table 1, and the viscosity of both sets of mixtures were determined using a Brookfield RV Viscometer. Samples were transferred to a 600 mL beaker and equilibrated in a water bath at 40°C overnight to ensure thermal uniformity. The spindle was centered in the beaker and submerged to the immersion groove for viscosity measurements. The spindle was then rotated at defined speeds to evaluate the shear-dependent viscosity of the formulation. In addition, hardness testing of the samples was performed using a TA XT2 Texture Analyzer.

**Table 1: Chewable fill material formulations**

| **Component** | **Formula 1 (% W/W)** | **Formula 2 (% W/W)** | **Formula 3 (% W/W)** | **Formula 4 (% W/W)** | **Formula 5 (% W/W)** | **Formula 6 (% W/W)** | **Formula 7 (% W/W)** | **Formula 8 (% W/W)** |
|---|---|---|---|---|---|---|---|---|
| Gelatin | 12.77 | 23.26 | 14.08 | 23.26 | 12.77 | 23.26 | 14.08 | 23.26 |
| Gelatin hydrolysate | 12.77 | 23.26 | 14.08 | 23.26 | 0 | 0 | 0 | 0 |
| Glucose Syrup | 0 | 0 | 0 | 0 | 12.77 | 23.26 | 14.08 | 23.26 |
| Sorbitol | 30.52 | 23.26 | 28.17 | 0.00 | 30.52 | 23.26 | 28.17 | 0.00 |
| Glycerol | 12.77 | 0.00 | 14.08 | 23.26 | 12.77 | 0.00 | 14.08 | 23.26 |
| Citric acid | 3.03 | 2.33 | 1.41 | 2.33 | 3.03 | 2.33 | 1.41 | 2.33 |
| Water* | Water was adjusted to a concentration of approximately 28% by weight. | | | | | | | |

| Appearance | yellow translucent gel | phase separation, yellow bottom, white top | yellow translucent gel | phase separation, yellow bottom, white top | yellow translucent gel | yellow translucent gel | yellow translucent gel | yellow translucent gel |
|---|---|---|---|---|---|---|---|---|
| Viscosity at 40°C (cP) | 540 | 26500 | 1250 | 27600 | 450 | 4850 | 848 | 3240 |
| Hardness at RT (g) | 39 | 127 | 53 | 79 | 40 | 99 | 53 | 109 |

Formulas 2 and 3 which contained gelatin hydrolysate were observed to exhibit phase separation characterized by undissolved gelatin hydrolysate migrating to the surface. Furthermore, the viscosity of these formulations exceeded the threshold for successful gummy-gel encapsulation via the rotary die process. In contrast, formulations 5-8, which contained the crystallization inhibitor, glucose syrup, achieved a comparable hardness and satisfactory mouthfeel, while maintaining a viscosity profile suitable for rotary die processing.

Thus, the addition of a crystallization inhibitor, such as glucose syrup, to the chewable fill mixture achieved optimal viscosity for rotary die encapsulation, while maintaining the desirable hardness and satisfactory mouthfeel.

### Example 6: Stability of chewable fill material formulations

In order to test the stability of the active ingredient in a gummy-gel formulation, fill mixtures containing the active ingredient Triprolidine HCl were prepared according to Table 2.

**Table 2: Fill mixture formulations**

| **Component No** | **Component** | **Formula 1** | | **Formula 2** | |
|---|---|---|---|---|---|
| | | **%W/W** | **%W/V** | **%W/W** | **%W/V** |
| 1 | Corn Syrup | 44.44 | 59.91 | 44.44 | 55.60 |
| 2 | Water | 8.89 | 11.98 | 8.89 | 11.12 |
| 3 | Hydrogenated Starch Hydrolysate | 0.00 | 0.00 | 23.67 | 29.61 |
| 4 | Sugar | 23.67 | 31.90 | 0.00 | 0.00 |
| 5 | Malic acid | 1.33 | 1.80 | 1.33 | 1.67 |
| 6 | Triprolidine HCl | 0.93 | 1.25 | 0.93 | 1.16 |
| 7 | Gelatin | 9.63 | 12.98 | 9.63 | 12.05 |
| 8 | Water | Water was adjusted to a concentration of approximately 11% by weight. | | | |

Formula 1 was prepared according to conventional gummy preparation methods which involved heating the mixture at temperatures of 65 °C and 120 °C, while Formula 2 was mixed at lower temperatures (< 65 °C), as set forth in Table 3.

**Table 3: Process for preparation of fill mixtures**

| **Process** | **Formula 1** | **Formula 2** |
|---|---|---|
| 1 | Mix 1, 2 and 4 at 85°C till dissolved (Mixture A) | Mix 1, 2, 3 and 5 at 55°C till dissolved (Mixture A) |
| 2 | Premix 7 and 8 at 65°C | Premix 7 and 8 at 65°C |
| 3 | Increase temperature to 120°C for 30 min | Add 6 to Mixture A at 55°C (Mixture B) |
| 4 | Cool to 80°C to add 5 and 6 to Mixture A (Mixture B) | Add premixed 7 and 8 to Mixture B, mix well |
| 5 | Add premixed 7 and 8 to Mixture B, mix well | - |

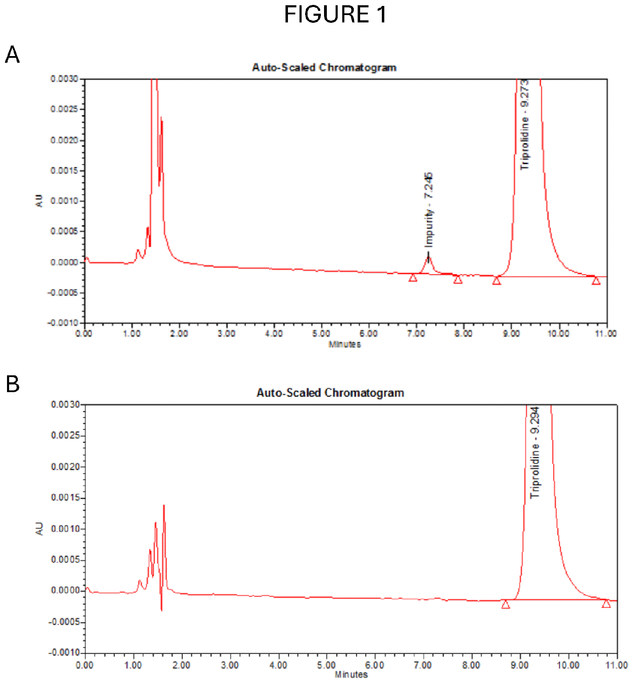

The active ingredient in the two mixtures was detected using High Performance Liquid Chromatography (HPLC). As shown in Figure 1A and Figure 1B, formula 1 showed the presence of 0.9% of degradation impurity of triprolidine HCl, while no degradation product was detected in Formula 2.

Thus, the preparation of chewable fill formulations at lower temperatures improves the stability of the active ingredient and reduces degradation when compared to conventional gummy preparation methods.

### Example 7: Stability of chewable gummy-gel formulations

To evaluate the long-term stability of active ingredients encapsulated in gummy gel formulations, fill mixtures containing the active ingredient Ubiquinol were prepared according to Table 4.

**Table 4: Fill mixture formulations**

| **Component** | **Formula 1** | | **Formula 2** | |
|---|---|---|---|---|
| | **%W/W** | **%W/V** | **%W/W** | **%W/V** |
| Corn Syrup | 45.5 | 58.4 | 45.5 | 55.7 |
| Water | 6.8 | 8.8 | 0.6 | 0.7 |
| Hydrogenated Starch Hydrolysate | - | - | 25.6 | 31.4 |
| Sugar | 25.6 | 32.8 | - | - |
| Malic acid | 0.5 | 0.6 | 0.5 | 0.6 |
| Ubiquinol | 5.7 | 7.3 | 5.7 | 7.0 |
| MCT | - | - | 6.3 | 7.7 |
| Gelatin | 7.4 | 9.5 | 7.4 | 9.1 |
| Water | Water was adjusted to a concentration of approximately 9% by weight. | | | |

Formula 1 was prepared according to conventional gummy preparation methods which involve heating the mixture at temperatures of 65 °C and 120 °C, while formula 2 was mixed at lower temperatures (< 65 °C) and encapsulated in a gel capsule using a rotary die process, as set forth in Table 5.

**Table 5: Process for preparation of fill mixtures**

| **Process** | **Formula 1** | **Formula 2** |
|---|---|---|
| 1 | Mix 1, 2 and 4 at 85°C till dissolved (Mixture A) | Mix 1, 2, 3 and 5 at 55°C till dissolved (Mixture A) |
| 2 | Premix 8 and 9 at 65°C | Premix 8 and 9 at 65°C |
| 3 | Increase the temperature to 120°C for 30 min | Add 6 and 7 to Mixture A at 55°C (Mixture B) |
| 4 | Cool to 80°C to add 5, 6 and 7 to Mixture A (Mixture B) | Add premixed 8 and 9 to Mixture B, mix well. |
| 5 | Add premixed 8 and 9 to Mixture B, mix well. place in a gummy mold and cool to room temperature. | Encapsulate into gummy-gel using a Rotary Die Process |

Following preparation, both samples were stored in open containers and exposed to ambient light and atmosphere. The levels of the impurity Coenzyme Q10 (CoQ10), an oxidation product of ubiquinol, were measured at the time of preparation (T0) and after 17 days (T17). As shown in Table 6, the levels of impurities in Formula 1 were higher than Formula 2 at both T0 and T17. Moreover, the increase in impurity levels over 17 days was over 4-fold higher than the increase in impurity levels for Formula 2.

**Table 6: Impurity levels in fill materials**

| **Parameter** | **Formula 1** | **Formula 2** |
|---|---|---|
| % Impurity at T0 | 10.03 | 6.95 |
| % Impurity at T17 | 16.30 | 8.48 |
| % Impurity increased from T0 to T17 | 6.27 | 1.53 |

Thus, the lower temperature preparation of a gummy gel mixture and its subsequent encapsulation in a softgel capsule improves the long-term stability of the active ingredient contained in the gummy gel.

### Example 8: Incorporation of lipid in gummy-gel capsules improves mouth feel

To evaluate the effect of lipid-based excipients in gummy gel capsules, gummy gel mixtures were prepared either with or without medium chain triglycerides (MCT) oil, as set forth in Table 7.

**Table 7: Fill mixture formulations**

| **Component** | **Formula 1** | | **Formula 2** | |
|---|---|---|---|---|
| | **%W/W** | **%W/V** | **%W/W** | **%W/V** |
| Gelatin | 12.00 | 14.7 | 12.00 | 14.4 |
| Glycerin | 50.00 | 61.3 | 50.00 | 60.0 |
| MCT | 0.00 | - | 10.00 | 12.0 |
| Hydrogenated Starch Hydrolysate | 16.00 | 19.6 | 6.00 | 7.2 |
| Citric acid | 2.00 | 2.4 | 2.00 | 2.4 |
| Water | Water was adjusted to a concentration of approximately 20% by weight. | | | |

The mixtures of Formula 1 and Formula 2 were prepared and encapsulated via a rotary die process. The resulting gummy-gels were stored under ambient conditions for a period of 14 days. Quantitative moisture analysis was performed using a thermogravimetric moisture analyzer (moisture balance) to determine the total volatile content as a percentage of mass.

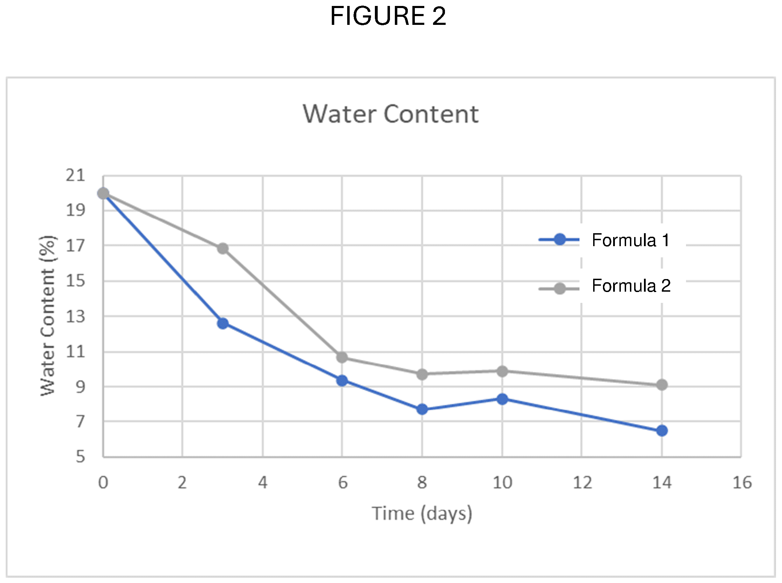

The moisture migration kinetics of the two samples over a period of 14 days is shown in Figure 2. Formula 1 which did not contain MCT oil exhibited a more rapid decline in water content, suggesting a lower affinity for moisture retention within the polymer matrix. In contrast, Formula 2 which was MCT-infused, maintained a consistently higher equilibrium moisture content throughout the 14-day study.

Thus, the data suggest that inclusion of MCT oil in Formulation 2 functions as a secondary plasticizing stabilizer or moisture-barrier regulator, by maintaining a higher internal water activity relative to Formulation 1. This retention of moisture prevents the chewable fill material from becoming overly brittle or hardened during storage, which is critical for the shelf-life stability of a chewable delivery system.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A chewable gel composition comprising a chewable fill material encapsulated by a chewable gel shell, wherein the chewable fill material comprises one or more gummy-gel forming agents present at a total of about 0.1-40% (w/w) of the chewable fill material.

2. The chewable gel composition of claim 1, wherein
the one or more gummy-gel forming agents is present at a total of about 0.1%-25%, 0.1%-20%, 0.1%-15%, 0.1%-13%, 2%-25%, 2%-20%, 5%-25%, 5%-24%, 6%-24%, 7%-24%, 2%-15%, 2%-13%, 4%-15%, 4%-13%, 5%-15%, 5%-13%, 7%-15%, 7%-13%, 8%-15%, or 8%-13% (w/w) of the chewable fill material; and/or
the one or more gummy-gel forming agents comprises a monomer, an oligomer, a polymer, a copolymer or combinations thereof; optionally wherein the one or more gummy-gel forming agents comprises gelatin, starch, pectin, carbomer, poloxamer or a combination thereof; further optionally wherein the one or more gummy-gel forming agents comprises gelatin.

3. The chewable gel composition of claim 1 or 2, wherein the chewable fill material further comprises one or more crystallization inhibitors.

4. The chewable gel composition of claim 3, wherein
the one or more crystallization inhibitors comprises glucose syrup, corn syrup, povidone, fructose syrup, glycerin, hydrogenated starch hydrolysate or a combination thereof; and/or
the one or more crystallization inhibitors are present at a total of about 30-90% (w/w) of the chewable fill material; optionally wherein the one or more crystallization inhibitors are present at a total of about 30%-85%, 30%-80%, 30%-70%, 40%-75%, 30%-60%, 35%-60%, 40%-60% or 40%-55%, (w/w) of the chewable fill material.

5. The chewable gel composition of any one of the preceding claims, wherein the chewable fill material further comprises one or more pH adjusting agents; optionally wherein
the one or more pH adjusting agents are present at a total of about 0.01-10% (w/w) or 0.1-5% (w/w) of the chewable fill material; further optionally wherein the one or more pH adjusting agents are present at a total of about 0.1-3%, 0.2-3%, or 0.2-2% (w/w) of the chewable fill material; and/or
the one or more pH adjusting agents comprise citric acid, ascorbic acid, lactic acid, sodium citrate, sodium bicarbonate, malic acid, or combinations thereof; further optionally wherein the one or more pH adjusting agents comprise at least one of citric acid or malic acid.

6. The chewable gel composition of any one of the preceding claims, wherein the chewable fill material further comprises one or more sweeteners; optionally wherein
the one or more sweeteners are present at a total of about 0.1-35% (w/w) of the chewable fill material; further optionally wherein the one or more sweeteners are present at a total of about 4-34%, 5-33%, 5-32%, or 5-20% (w/w) of the chewable fill material; and/or
the one or more sweeteners comprise sucrose, glycerin, glucose, hydrogenated starch hydrolysate, sucralose, xylitol, or combinations thereof.

7. The chewable gel composition of any one of the preceding claims, wherein the chewable fill material further comprises one or more flavoring agents, optionally wherein
the one ore more flavoring agents are present at a total of about 0.1-5% (w/w) of the chewable fill material; further optionally wherein the one or more flavoring agents are present at a total of about 0.5-3%, 0.7-2%, or 1-1.6% (w/w) of the chewable fill material; and/or
the one ore more flavoring agents comprise natural flavors and/or artificial flavors.

8. The chewable gel composition of any one of the preceding claims, wherein the chewable fill material further comprises one or more solvents, optionally wherein
the one or more solvents are present at a total of about 0.1-15% (w/w) of the chewable fill material; further optionally wherein the one or more solvents are present at a total of about 0.5-13%, 1-12%, or 1.5-10% (w/w) of the chewable fill material; and/or
the one or more solvents comprises water, oil, propylene glycol, polyethylene glycol (PEG), medium-chain triglycerides (MCT) oil, or combinations thereof.

9. The chewable gel composition of any one of the preceding claims, wherein the chewable fill material further comprises one or more lipids; optionally wherein
the one or more lipids are present at a total of about 0.1-20% (w/w) of the chewable fill material; further optionally wherein the one or more lipids are present at a total of about 0.5-15%, 2-15%, 5-15%, or 5-12% (w/w) of the chewable fill material; and/or
the one or more lipids comprises MCT oil, olive oil, sunflower oil, fish oil, krill oil, flaxseed oil, hemp oil, chia oil, evening primrose oil, borage oil, black cumin oil, avocado oil, eicosapentaenoic acid (EPA), docosahexaenoic acid (DHA), beeswax, carnauba wax, rice bran, candelilla, or combinations thereof; further optionally wherein the one or more lipids comprises MCT oil.

10. The chewable gel composition of any one of the preceding claims, wherein the chewable fill material further comprises an active ingredient, optionally wherein the active ingredient is a pharmaceutical compound, a nutraceutical compound, or a dietary supplement, further optionally wherein the active ingredient comprises dextromethorphan HBr, doxylamine succinate, diphenhydramine HCl, ubiquinol, creatine, lutein, zeaxanthin, ibuprofen, guaifenesin, acetaminophen, triprolidine HCl, CoQ10, or combinations thereof.

11. The chewable gel composition of any one of the preceding claims, wherein
the chewable fill material is a gummy composition;
the chewable fill material has a viscosity of about 300-16,000 cP at a temperature of about 35-45°C, optionally at 40 °C, prior to encapsulation by the chewable gel shell; optionally wherein the chewable fill material has a viscosity of about 350-15,000 cP, 350-12,500 cP, 400-10,000 cP, 400-9,000 cP, 400-8,000 cP, 500-8,000 cP, 400-7,000 cP, 400-6,000 cP, 400-7,000 cP, 400-6,000 cP, 400-5,000 cP, 400-4,000 cP, 400-3,000 cP, 400-2,000 cP, 400-1,000 cP, or 450-900 cP at a temperature of about 40 °C, prior to encapsulation by the chewable gel shell; further optionally wherein encapsulation is through a rotary die process; and/or
the chewable gel shell comprises one or more of gelling agents, plasticizers, anti-tacking agents, softening agents, sweeteners, flavoring agents, colorants, preservatives or combinations thereof.

12. A chewable gel composition comprising a chewable fill material encapsulated by a chewable gel shell, wherein the chewable fill material comprises:
3-15% (w/w) gelatin, optionally about 6-13% (w/w) gelatin; and
40-90% (w/w) of one or more crystallization inhibitors, optionally wherein the crystallization inhibitor is selected from the group consisting of corn syrup, glycerin, glucose syrup, fructose syrup, hydrogenated starch hydrolysate and a combination thereof.

13. The chewable gel composition of claim 12, wherein
the chewable fill material further comprises about 0.1-3% (w/w) of one or more pH adjusting agents; optionally wherein the pH adjusting agent is at least one of malic acid or citric acid;
the chewable fill material further comprises povidone, propylene glycol, xylitol and/or a flavoring agent;
the chewable fill material further comprises about 5-15% (w/w) MCT oil; and/or
the chewable fill material has a viscosity of about 300-16,000 cP at a temperature of about 35-45°C, optionally at 40 °C, prior to encapsulation by the chewable gel shell.

14. A method of making a chewable gel composition, comprising:
(a) preparing a chewable fill material mixture comprising one or more gummy-gel forming agents, one or more crystallization inhibitors and a pH adjusting agent;
(b) incubating the mixture of step (a) at a fixed temperature for a time period sufficient to achieve a uniform mixture; and
(c) encapsulating the uniform mixture of step (b) by a chewable gel shell using a rotary die process.

15. The method of claim 14,
wherein step (b) comprises incubating the mixture at a temperature ranging from 25-70 °C;
wherein step (b) comprises maintaining the mixture at a fixed temperature for a time period of about 0.5-48 hours; and/or
wherein the uniform mixture of step (b) has a viscosity of about 300-16,000 cP at a temperature of about 35-45°C, optionally at 40 °C; optionally wherein the uniform mixture of step (b) has a viscosity of about 350-15,000 cP, 350-12,500 cP, 400-10,000 cP, 400-9,000 cP, 400-8,000 cP, 500-8,000 cP, 400-7,000 cP, 400-6,000 cP, 400-7,000 cP, 400-6,000 cP, 400-5,000 cP, 400-4,000 cP, 400-3,000 cP, 400-2,000 cP, 400-1,000 cP, or 450-900 cP at a temperature of about 40 °C.
